# FASCICULE DE BREVET EUROPEEN

(11) **EP 1 457 194 B1**
(45) Date de publication et mention de la délivrance du brevet: **26.11.2008**
(21) Numéro de dépôt: 04290370.8
(22) Date de dépôt: 12.02.2004
(51) Int. Cl.: A61K 8/23, A61K 8/26, A61K 8/73, A61K 8/81, A61K 8/84, A61Q 5/02

(54) **Compositions cosmétiques comprenant un copolymère d' acide méthacrylique, des particules minérales insolubles et un polymère cationique ou amphotère et leurs utilisations**
Kosmetische Zusammensetzungen enthaltend ein Methacrylsäure-Copolymer, ein kationisches oder amphiphiles Polymer und unlösliche Mineralpartikel sowie ihre Verwendung
Cosmetic compositions comprising a methacrylic acid copolymer, insoluble mineral particles and a cationic or amphoteric polymer and their use

(30) Priorité: 11.03.2003 FR 0302997
(43) Date de publication de la demande: 15.09.2004
(73) Titulaire: L'ORÉAL, 75008 Paris (FR)
(72) Inventeur: Maubru, Mireille, CHATOU (FR); Restle, Serge, 95390 Saint-Prix (FR); Perron, Béatrice, 78350 Jouy en Josas (FR)
(74) Mandataire: Le Blainvaux Bellegarde, Françoise

(56) Documents cités:
- EP-A- 1 210 932
- WO-A-01/74311
- WO-A-01/76552
- WO-A-98/18431
- DE-A- 10 163 500
- GB-A- 2 315 215
- ANOM.: "New gels and thickeners for cosmetics: structure, application, formulation" SOFW JOURNAL, vol. 128, no. 5, 2002, pages 16-18,20, XP009021303

## Description

La présente invention concerne de nouvelles compositions cosmétiques comprenant dans un milieu cosmétiquement acceptable au moins un copolymère acide méthacrylique / acrylate d'alkyle en C₁-C₄ réticulé, au moins un polymère cationique ou amphotère et au moins des particules minérales solides insolubles insoluble dans l'eau choisies parmi les argiles, les particules essentiellement constituées d'alumine, les particules essentiellement constituées de carbonate de calcium et le sulfure de sélénium.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou amphotères ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).

En outre, l'usage des polymères cationiques ou amphotères dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

Il a déjà été proposé l'utilisation de particules dans des compositions rincées, de façon à améliorer le toucher et l'aspect des cheveux. A titre d'illustration, le brevet US 5 334 376 propose l'ajout de particules de carbonate de calcium dans des compositions de conditionnement des cheveux comprenant une silicone, un alcool gras et une amide.

Dans la demande de brevet DE 199 46 784, il a également été proposé l'utilisation de particules de différents oxydes, hydroxydes, carbonates, silicates ou phosphates, dans des compositions capillaires, pour réduire l'aspect gras des cheveux. Il est prévu, en toutes généralités, d'associer ces particules aux ingrédients classiques des shampooings.

Cependant, on a constaté que ces particules conduisaient à la formation d'une couche inesthétique à la surface du shampooing nuisible aux performances du shampooing. Pour éviter l'apparition de ce phénomène, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

En résumé, il s'avère que les compositions cosmétiques actuelles comprenant des polymères cationiques ou amphotères, ne donnent pas encore complètement satisfaction.

On connaît dans l'état de la technique des compositions cosmétiques en particulier détergentes comprenant un copolymère d'acide méthacrylique et d'acrylate d'alkyle comme agent de stabilisation ou de suspension d'ingrédients insolubles dans l'eau comme des silicones ou des corps gras. De telles compositions ont été décrites notamment dans la demande de brevet WO01/76552. Les qualités de mousse et les propriétés cosmétiques obtenues avec ces compositions ne sont pas encore suffisamment satisfaisantes.

La demanderesse a maintenant découvert que l'association d'un copolymère réticulé acide méthacrylique / acrylate d'alkyle en C₁-C₄, d'un polymère cationique ou amphotère et des particules minérales particulières permettait de remédier à ces inconvénients.

En effet, il a été constaté que l'utilisation dudit copolymère acrylique dans les compositions de la présente invention permettait d'obtenir sur les matières kératiniques, notamment les cheveux de très bonnes propriétés cosmétiques particulièrement au niveau de la légèreté, de la douceur, du lissage au toucher, au niveau de la souplesse et de la malléabilité sur cheveux séchés. Il a aussi été constaté que les compositions de l'invention permettaient d'apporter de la texture aux cheveux (sensation d'épaisseur accrue) et une meilleure tenue de la coiffure.

Par ailleurs, les compositions selon l'invention sont stables et présentent un aspect visuel esthétique. Les propriétés d'usage (aspect, consistance, abondance de la mousse, élimination de la mousse) sont très satisfaisantes.

Les compositions de l'invention appliquées sur la peau notamment sous forme de bain moussant ou de gel douche, apportent une amélioration de la douceur de la peau.

Ainsi, la présente invention a pour objet de nouvelles compositions cosmétiques, comprenant, dans un milieu cosmétiquement acceptable, au moins un copolymère d'acide méthacrylique et d'acrylate d'alkyle en C₁-C₄ réticulé, au moins un polymère cationique ou amphotère et au moins des particules minérales solides insolubles dans l'eau choisies parmi les argiles, les particules essentiellement constituées d'alumine, les particules comprenant au moins 10% en poids de carbonate de calcium et le sulfure de sélénium.

L'invention a également pour objet l'utilisation d'une composition telle que définie ci-dessus pour apporter de la souplesse aux cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.

Un autre objet de l'invention concerne l'utilisation d'un copolymère réticulé acide méthacrylique / acrylate d'alkyle en C₁-C₄ dans, ou pour la fabrication d'une composition cosmétique comprenant un polymère cationique ou amphotère et au moins des particules minérales solides insolubles dans l'eau choisies parmi les argiles, les particules essentiellement constituées d'alumine, les particules comprenant au moins 10 % en poids de carbonate de calcium et le sulfure de sélénium.

Par insolubles dans l'eau, on comprend selon l'invention les composés insolubles dans l'eau à une concentration supérieure ou égale à 0,1% en poids dans l'eau à 25°C, c'est à dire qu'ils ne forment pas une solution macroscopiquement isotrope et transparente.

Les différents objets de l'invention vont maintenant être détaillés. L'ensemble des significations et définitions des composés utilisés dans la présente invention données ci-dessous sont valables pour l'ensemble des objets de l'invention.

L'une des caractéristiques essentielles de l'invention est la présence d'un copolymère d'acide méthacrylique et d'acrylate d'alkyle en C₁-C₄ , réticulé.

L'acide méthacrylique est présent de préférence dans des quantités allant de 20 à 80% en poids et plus particulièrement de 25 à 70 % en poids et encore plus particulièrement de 35 à 60% en poids par rapport au poids total du copolymère.

L'acrylate d'alkyle est présent de préférence dans des quantités allant de 15 à 80% en poids et plus particulièrement de 25 à 75 % en poids et encore plus particulièrement de 40 à 65% en poids par rapport au poids total du copolymère. Il est choisi notamment parmi l'acrylate de méthyle, l'acrylate d'éthyle ou l'acrylate de butyle et plus particulièrement l'acrylate d'éthyle.

Ce copolymère est partiellement ou totalement réticulé par au moins un agent réticulant classique. Les agents réticulants sont notamment des composés polyinsaturés, en particulier éthylèniquement polyinsaturés. Ces composés sont notamment les polyalcényléthers de sucrose ou de polyols, les diallylphtalates, le divinylbenzene, le (méth)acrylate d'allyle, di(méth)acrylate d'éthylèneglycol, le méthylène bis-acrylamide, le tri(méth)acrylate de triméthylol propane, l'itaconate de diallyle, le fumarate de diallyle, le maléate de diallyle, le (méth)acrylate de zinc, les dérivés d'huile de ricin ou de polyols fabriqués à partir d'acides carboxyliques insaturés.
Comme agent réticulant, on peut également utiliser des composés monomères insaturés et comportant un groupe réactif susceptible de réagir avec une insaturation pour former un copolymère réticulé.
La teneur en agent réticulant varie en général de 0,01 à 5% en poids et de préférence de 0,03 à 3% en poids et encore plus particulièrement de 0,05 à 1% en poids par rapport au poids total du copolymère.

Selon une forme particulièrement préférée, le copolymère de l'invention peut se présenter notamment sous forme de dispersion dans l'eau. La taille moyenne en nombre des particules de copolymère dans la dispersion est généralement comprise entre 10 et 500 nm et de préférence entre 20 et 200 nm et plus préférentiellement de 50 à 150 nm.

Ces copolymères sont notamment décrit dans la demande WO01/76552.

On utilisera plus particulièrement le copolymère acide méthacrylique/acrylate d'éthyle réticulé sous forme de dispersion aqueuse à 30% de matière active fabriqué et vendu sous le nom CARBOPOL AQUA SF-1 par la société NOVEON.

La concentration en copolymère est généralement comprise entre 0,01 et 10% en poids par rapport au poids total de la composition et de préférence entre 0,1 et 5% en poids.

Les particules selon l'invention peuvent avoir une forme quelconque, par exemple la forme de sphères, de paillettes, d'aiguilles, de plaquettes ou des formes totalement aléatoires.
Au sens de la présente invention, on entend par "taille primaire de particule", la dimension maximale qu'il est possible de mesurer entre deux points diamétralement opposés d'une particule individuelle. La taille peut être déterminée par microscopie électronique à transmission ou à partir de la mesure de la surface spécifique par la méthode BET.

Les argiles peuvent être d'origine naturelle ou synthétique, mais de préférence naturelle.

Les compositions conformes à l'invention peuvent bien entendu contenir une ou plusieurs argiles.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

A titre d'exemples de tels produits, on peut citer les argiles de la famille de la kaolinite telles que la kaolinite, la dickite, la nacrite, les argiles de la famille de l'halloysite, de la dombassite, de l'antigorite, de la benthiérine, de la pyrophyllite, des montmorillonites, de la beidellite, des vermiculites, du talc, de la stévensite, des hectorites, des saponites, des chlorites, de la sépiolite.

Les argiles peuvent également être modifiées chimiquement par divers composés tels que les acides acryliques, les polysaccharides (par exemple la carboxyméthylcellulose) ou les cations organiques.

Selon un mode particulièrement préféré de réalisation de la présente invention, l'argile mise en oeuvre est choisie parmi la kaolinite, les montmorillonites, les hectorites.

Le sulfure de sélénium utilisé conformément à la présente invention peut comporter un atome de sélénium pour deux atomes de soufre. Il peut également avoir une structure cyclique SexSy dans laquelle x+y=8.

Le disulfure de sélénium utilisable selon l'invention est une poudre dont les particules ont une taille moyenne en nombre inférieure à 200 microns et de préférence inférieure à 25 microns.

Les particules essentiellement constituées d'alumine selon l'invention comprennent au moins 70 % en poids d'alumine, de préférence plus de 90 % en poids d'alumine. Elles peuvent présenter une taille primaire moyenne en nombre comprise entre 2 et 200 nm, et de préférence entre 5 et 50 nm.
Les particules d'alumine selon l'invention sont essentiellement constituées par une quelconque alumine éventuellement hydratée telle que, par exemple, la boehmite.

Les particules comprenant au moins 10% en poids de carbonate de calcium selon l'invention comprennent de préférence au moins 70 % en poids de carbonate de calcium, de préférence plus de 90 % en poids de carbonate de calcium. Elles peuvent présenter une taille primaire moyenne en nombre comprise entre 2 nm et 2 microns, et de préférence entre 5 et 500 nm, plus préférentiellement encore entre 10 et 250 nm.

Conformément à la présente invention, les particules peuvent être des particules massiques formées entièrement de carbonate de calcium. Le carbonate de calcium peut également constituer totalement ou partiellement le coeur de la particule, ce dernier étant recouvert d'un autre constituant, comme par exemple, un oxyde, un silicate ou un métal. Le carbonate de calcium peut encore former uniquement le revêtement d'un substrat de constitution chimique différente, comme par exemple un oxyde, un silicate ou un métal.

Dans le cas où les particules sont formées par du carbonate de calcium et d'autres charges, le carbonate de calcium se trouve à l'état libre et ne forme pas de liaisons chimiques avec les autres charges. Il s'agit alors d'un alliage entre le carbonate de calcium et d'autres charges, notamment avec des oxydes de métaux ou de métalloïdes, en particulier obtenu par fusion thermique de ces différents constituants.

Lorsque les particules comprenant au moins 10 % en poids de carbonate de calcium comprennent, en outre, un oxyde de métal ou de métalloïde, celui-ci est notamment choisi parmi l'oxyde de silicium, de bore ou d'aluminium.

De préférence, les particules contiennent au moins 50 % en poids de carbonate de calcium, mieux encore au moins 70 % en poids, et les particules constituées à plus de 90 % en poids de carbonate de calcium sont particulièrement préférées selon la présente invention.

Plus avantageusement encore, les particules comprenant au moins 10 % en poids de carbonate de calcium, sont des particules de carbonate de calcium substantiellement pur.

Le carbonate de calcium convenant dans les compositions de la présente invention peut être d'origine naturelle ou peut être d'origine synthétique. Dans ce dernier cas, il peut être obtenu à partir d'oxyde de calcium, de peroxyde de calcium, d'acétate ou d'éthylate de calcium.

Les particules minérales solides insolubles sont utilisées de préférence en une quantité comprise entre 0,001 et 20% en poids par rapport au poids total de la composition. Plus préférentiellement, cette quantité est comprise entre 0,005 et 15% en poids par rapport au poids total de la composition et encore plus préférentiellement entre 0,01 et 10% en poids.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes: 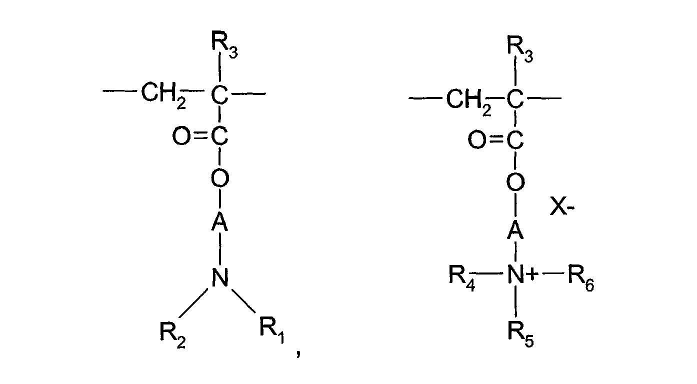 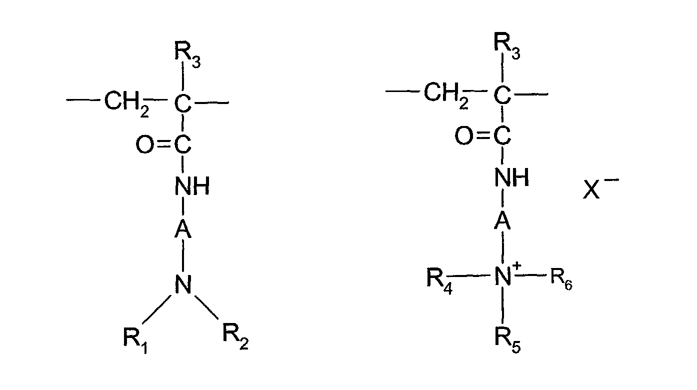 dans lesquelles:
R₃ , identiques ou différents, désignent un atome d'hydrogène ou un radical CH₃;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
R₁ et R₂, identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymères vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.

Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "Celquat L 200" et "Celquat H 100" par la Société National Starch.

Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR C13 S, JAGUAR C 15, JAGUAR C 17 ou JAGUAR C162 par la société RHODIA.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(7) les cyclopolymères d'alkyl diallyl amine ou de dialkyl diallyl ammonium tels que les homopolymères ou copolymères comportant comme constituant principal de la chaîne des motifs répondant aux formules (II) ou (III) : 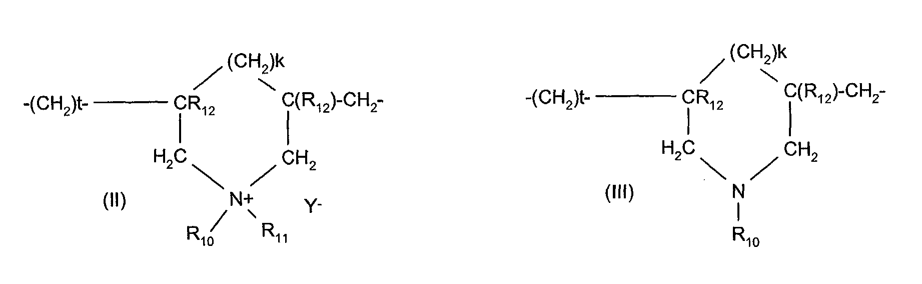 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide commercialisés sous la dénomination "MERQUAT 550".

(8) les polymères de diammonium quaternaire comprenant des motifs récurrents répondant à la formule : 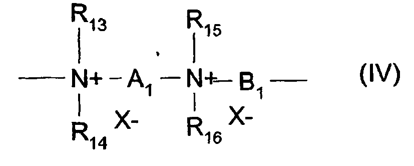 formule (IV) dans laquelle :
R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :

   -(CH2-CH2-O)x-CH2-CH2-

   -[CH2-CH(CH3)-O]y-CH2-CH(CH3)-

   où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

   -CH2-CH2-S-S-CH2-CH2- ;
d) un groupement uréylène de formule : -NH-CO-NH- ;

De préférence, X⁻ est un anion tel que le chlorure ou le bromure.

Ces polymères ont une masse molaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule : 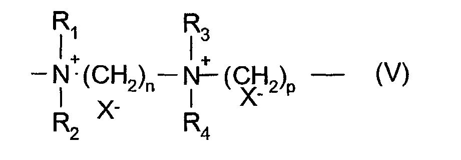 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (V) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).

(9) les polymères de polyammonium quaternaires comprenant des motifs de formule (VI): 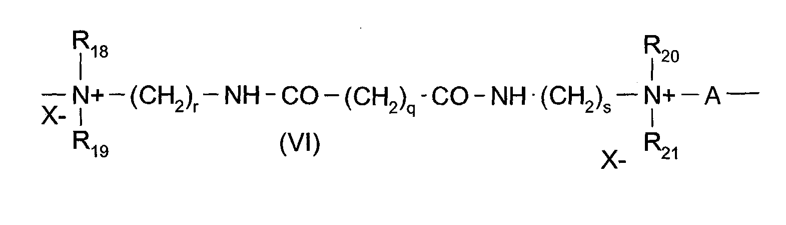 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X⁻ désigne un anion tel qu'un halogènure,
A désigne un radical d'un dihalogénure ou représente de préférence -CH₂-CH₂-O-CH₂-CH₂-.

De tels composés sont notamment décrits dans la demande de brevet EP-A-122 324.

On peut par exemple citer parmi ceux-ci, les produits "Mirapol® A 15", "Mirapol® AD1 ", "Mirapol® AZ1" et "Mirapol® 175" vendus par la société Miranol.

(10) Les polymères quaternaires de vinylpyrrolidone et de vinylimidazole tels que par exemple les produits commercialisés sous les dénominations Luviquat® FC 905, FC 550 et FC 370 par la société B.A.S.F.

(11) Les polyamines comme le Polyquart® H vendu par COGNIS, référencé sous le nom de «POLYETHYLENEGLYCOL (15) TALLOW POLYAMINE » dans le dictionnaire CTFA.

(12) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Les polyalkylèneimines utilisées préférentiellement selon l'invention sont des polymères comprenant de 6 à 20.000 motifs de répétition. De préférence, on choisit des polyalkylène imines comprenant au moins 5% d'amines tertiaires, avantageusement au moins 10% de fonctions amines tertiaires, et encore plus préférentiellement au moins 20%. Ces polymères peuvent être des homopolymères ou des copolymères, linéaires, branchés ou de structures dendrimériques.

Ces polymères comprennent les motifs répétitifs suivants : 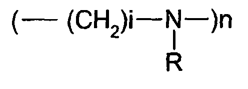 dans lesquels :
i représente un entier supérieur ou égal à 2 et de préférence inférieur ou égal à 6, préférentiellement i=2 ;
n représente un entier variant de 6 à 20.000 et de préférence de 8 à 2500
R représente un atome d'hydrogène ou un motif 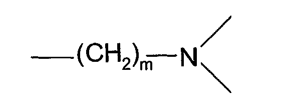
dans lequel m représente un entier supérieur ou égal à 2, préférentiellement m=2 ;

Ces polymères sont généralement terminés par des fonction amines terminales notamment des amines primaires.

Selon l'invention, les polyalkylèneimines sont de préférence des polyalkylène(C2-C4)imines et plus particulièrement des polyéthylèneimines.

Les polyalkylèneimines utilisées conformément à l'invention ont en général un poids moléculaire moyen en poids compris entre 300 et 100.000 et de préférence entre 350 et 50.000 et plus particulièrement entre 400 et 10.000.

Les poids moléculaires sont déterminés par diffusion quasi élastique de la lumière.

Les polyalkylèneimines ont de préférence une densité de charge cationique inférieure ou égale à 20 meq/g et, de préférence, supérieure ou égale à 0,05meq/g.
La densité de charge peut être déterminée selon la méthode Kjeldahl ou calculée.

Les polyalkylèneimines sont notamment décrites dans l'ouvrage "Polymer Science dictionary" 2ème édition, Mark ALGER, CHAPMAN & HALL, 1997.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, les polyalkylèneimines, en particulier les polyéthylèneimines et leurs mélanges.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes; K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué comprenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société COGNIS.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société NALCO.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide comprenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale : 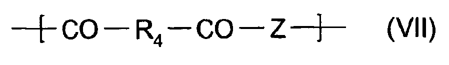 dans laquelle R₄ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical de formule (VIII) 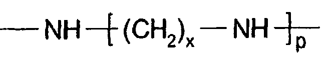 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (VIII) ci-dessus, dans lequel x=2 et p=1 et qui dérive de l'éthylènediamine, ou le radical dérivant de la pipérazine : 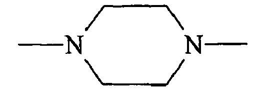
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule (IX): 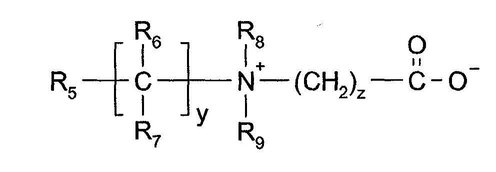 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle/méthacrylate de diméthyl-carboxyméthylammonio-éthyl tel que le produit vendu sous la dénomination DIAFORMER Z301 par la société SANDOZ.
(5) les polymères dérivés du chitosane comportant des motifs monomères répondant aux formules (X), (XI), (XII) suivantes : 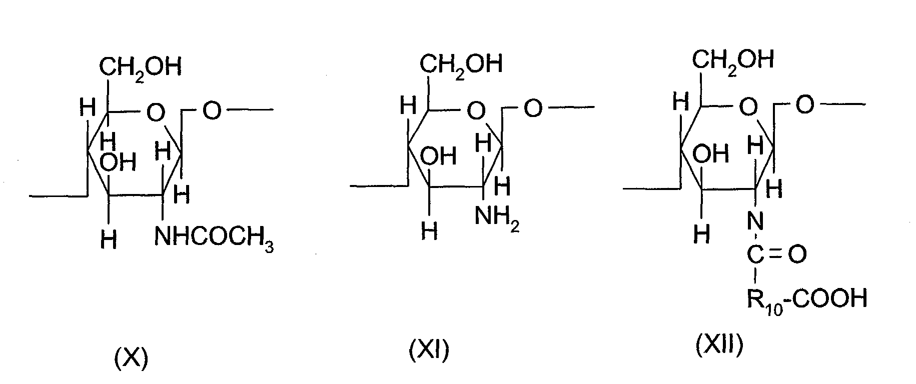 le motif (X) étant présent dans des proportions comprises entre 0 et 30%, le motif (XI) dans des proportions comprises entre 5 et 50% et le motif (XII) dans des proportions comprises entre 30 et 90%, étant entendu que dans ce motif (XII), R₁₀ représente un radical de formule : 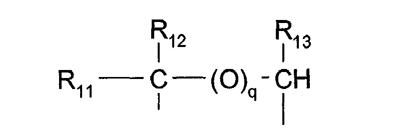 dans laquelle
   si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XIII) tels que ceux décrits par exemple dans le brevet français 1 400 366 : 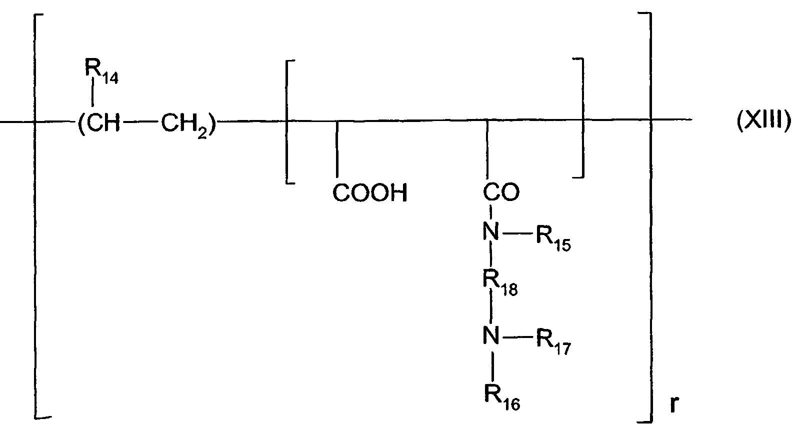 dans laquelle R₁₄ représente un atome d'hydrogène, un radical CH₃O CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂- , -CH₂-CH₂-CH₂- , -CH₂-CH(CH₃)- , R₁₆ ayant les significations mentionnées ci-dessus,
   ainsi que les homologues supérieurs de ces radicaux et comprenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      - D-X-D-X-D- (XIV)

      où D désigne un radical 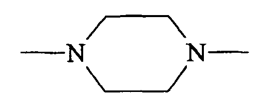 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      - D-X-D-X- (XV)
   où D désigne un radical 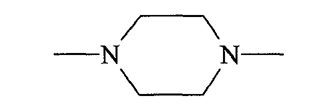 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1 ), notamment les copolymères de sel (par exemple halogénure) de diméthyldiallylammonium et d'acide acrylique.

Selon l'invention, le ou les polymères cationiques ou amphotères peuvent représenter de 0,001 % à 20 % en poids, de préférence de 0,01 % à 10% en poids et plus particulièrement de 0,1 à 5% en poids par rapport au poids total de la composition finale.

Selon un mode de réalisation particulier, les compositions selon l'invention comprennent en outre au moins une silicone ou un autre agent bénéfique pour les matières kératiniques en particulier les cheveux tels que notamment les esters d'acides carboxyliques en C1-C30 et d'alcools mono ou polyhydroxylés en C1-C30, les huiles végétales, animales, minérales ou de synthèse, les cires, les céramides, les pseudocéramides.

Les silicones additionnelles utilisables conformément à l'invention sont en particulier des polyorganosiloxanes insolubles dans la composition et peuvent se présenter sous forme d'huiles, de cires, de résines ou de gommes et les silicones organomodifiées.

Ces organopolysiloxanes additionnels sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones additionnelles sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 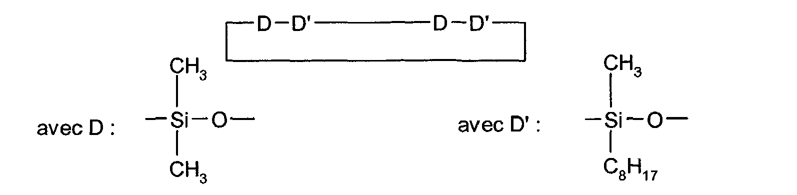 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

Ces silicones additionnelles sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société DEGUSSA qui sont des polyalkyl (C₁-C₂₀) siloxanes.

Les gommes de silicone utilisables en tant qu'additif sont notamment des polydiorganosiloxanes ayant des masses molaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,

Des produits plus particulièrement utilisables sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids molaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables en tant qu'additif sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 .
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 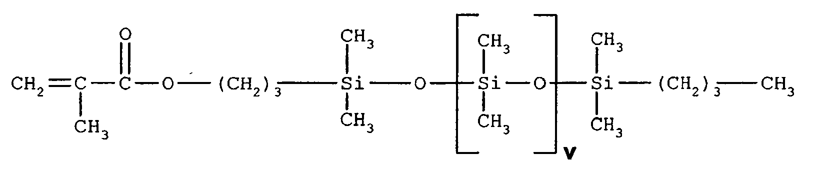
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent être utilisées tel quel ou sous forme de solutions, d'émulsions, de nanoémulsions ou de microémulsions.

Les silicones particulièrement préférés conformément à l'invention sont :
- les silicones non volatiles choisies dans la famille des polyalkylsiloxanes à groupements terminaux triméthylsilyle telles que les huiles ayant une viscosité comprise allant de 0,2 à 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polysiloxanes à groupements aminés tels que les amodiméthicones ou les triméthylsilylamodiméthicones ;

Selon l'invention, les silicones additionnelles ou les autres agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un agent tensioactif qui est généralement présent en une quantité comprise entre 0,01% et 50% en poids environ, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.
Parmi les alkyl (C₈-C₂₀) amidoalkyl (C₁-C₆) bétaïnes on peut citer la cocoamidopropylbétaïne vendue notamment par DEGUSSA sous le nom de TEGOBETAINE F50.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.

### (iv) Les tensioactifs cationiques peuvent être choisis parmi :

A) les sels d'ammonium quaternaires de la formule générale (XVI) suivante : 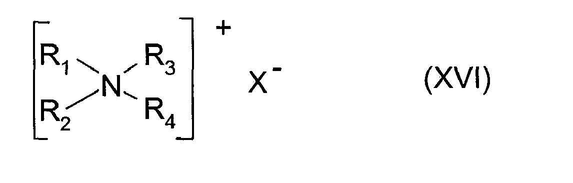 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XVII) suivante : 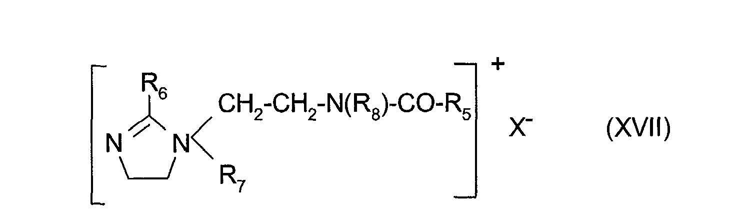 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997)
   ou le Quaternium-83 (CTFA 1997) commercialisés sous les dénominations "REWOQUAT" W 75, W90, W75PG, W75HPG par la société WITCO,
C) - les sels de diammonium quaternaire de formule (XVIII) : 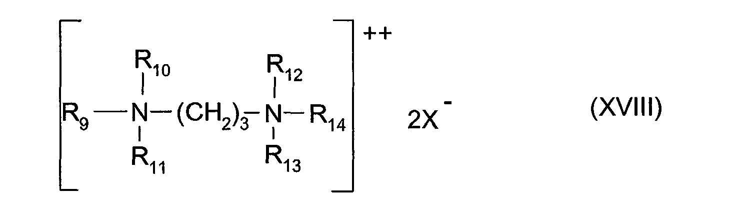 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire comprenant au moins une fonction ester de formule (XIX) suivante : 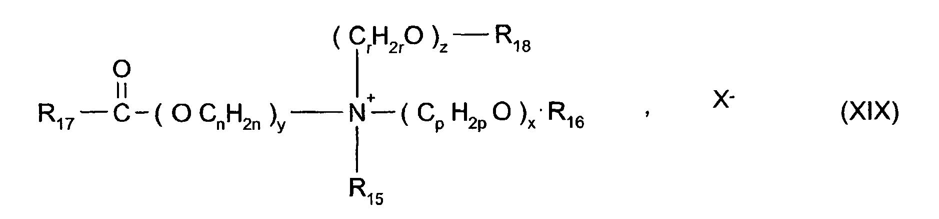 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 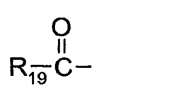
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 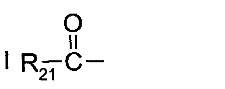
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XIX) dans laquelle:
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 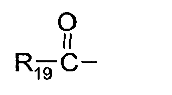
   - les radicaux méthyle, éthyle ou hydrocarbonés en C₁₄-C₂₂.
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 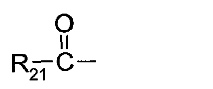
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et au moins un agent tensioactif amphotère.

La composition de l'invention peut également contenir au moins un additif choisi parmi les épaississants, les parfums, les agents nacrants, les conservateurs, les filtres solaires siliconés ou non, les polymères anioniques ou non ioniques non siliconés, les protéines non cationiques, les hydrolysats de protéines non cationiques, l'acide méthyl-18 eicosanoique, les hydroxyacides, les vitamines, les provitamines telles que le panthénol, et tout autre additif classiquement utilisé dans le domaine cosmétique qui n'affecte pas les propriétés des compositions selon l'invention.

Les compositions conformes à l'invention peuvent ainsi contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Le milieu physiologiquement et notamment cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol; les alkylèneglycols comme le propylèneglycol, les éthers de glycols.

De préférence, la composition comprend de 50 à 95 % en poids d'eau par rapport au poids total de la composition et plus particulièrement de 60 à 90% en poids.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins un tensioactif détergent.

Le ou les tensioactifs peuvent être indifféremment choisis, seuls ou en mélanges, au sein des tensioactifs anioniques, amphotères, non ioniques tels que définis ci-dessus.
Dans les compositions détergentes conformes à l'invention, on utilise de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.
Un mélange particulièrement préféré est un mélange comprenant au moins un agent tensioactif anionique et au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes ou les alkylamidobétaïnes et en particulier la cocobétaïne commercialisée sous la dénomination "DEHYTON AB 30" en solution aqueuse à 32 % de MA par la société COGNIS ou la cocoamidopropylbétaïne vendue notamment par DEGUSSA sous le nom de TEGOBETAINE F50.

La quantité et la qualité des tensioactifs sont celles suffisantes pour conférer à la composition finale un pouvoir moussant et/ou détergent satisfaisant.

Ces compositions détergentes sont de préférence moussante et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404/ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et comprenant 50 ml de la composition à tester. La mesure est faite 5 minutes après l'arrêt de l'écoulement de la composition.

Ainsi, selon l'invention, les tensioactifs détergents peuvent représenter de 3 % à 50 % en poids, de préférence de 6 % à 35 % en poids, et encore plus préférentiellement de 8 % à 25 % en poids, du poids total de la composition finale.

Les compositions détergentes peuvent également contenir des tensioactifs pour améliorer la mousse notamment des alcanoamides d'acide gras en C12-C20.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, sa concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des matières kératiniques en particulier les cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a réalisé le shampooing conforme à l'invention de composition suivante :

| Composition | Exemple 1 | |
|---|---|---|
| Lauryléthersulfate de sodium à 2.2 moles d'oxyde d'éthylène | 12,5 | g M.A. |
| Cocoamidoéthyl (N-hydroxyéthyl, N-carboxyméthyl) glycinate de sodium (MIRANOL C2M de RHODIA) | 2,5 | g M.A. |
| Copolymère réticulé acide méthacrylique / acrylate d'éthyle en émulsion aqueuse à 30% M.A., commercialisé sous la dénomination Carbopol Aqua SF1 par la société NOVEON | 3 | g M.A. |
| Oxyde d'aluminium (Aluminium oxid C de DEGUSSA-HÜLS) | 0,5 | g |
| Hydroxyéthylcellulose réticulée à l'épichlorhydrine, quaternisée par la triméthylamine (JR400 de AMERCHOL) | 0,5 | g |
| Conservateurs, parfum | q.s. | |
| Agent de pH q.s. | pH | 6,5 |
| Eau déminéralisée q.s.p. | 100 | g |

Les compositions sont stables. Les cheveux mouillés ne sont pas chargés et la mise en forme est aisée.

### EXEMPLE 2

On a réalisé le shampooing conforme à l'invention de composition suivante :

| | |
|---|---|
| Laurylsulfate de sodium | 12,5 gMA |
| Cocoamidopropylbétaine | 2,5 gMA |
| Polyéthylèneimine (LUPASOL de BASF) | 0,025 g |
| Carbonate de calcium | 5 g |
| Copolymère réticulé acide méthacrylique / acrylate d'éthyle en émulsion aqueuse à 30% M.A., commercialisé sous la dénomination Carbopol | |
| Aqua SF1 par la société NOVEON | 3 gMA |
| Conservateurs qs | |
| Eau déminéralisée qsp | 100 g |

### EXEMPLES 3 et 4

On a réalisé 2 shampooings conformes à l'invention de composition suivante :

| Composition | Exemple 3 | Exemple 4 |
|---|---|---|
| Lauryléthersulfate de sodium à 2.2 moles d'oxyde d'éthylène | 11.2 g M.A. | 15.5 g M.A. |
| Cocoylbétaïne | - | 2.4 g M.A. |
| | | |
| Cocoamidoéthyl (N-hydroxyéthyl, N-carboxyméthyl) glycinate de sodium (Miranol C2M de RHODIA) | 3.8 g M.A | - |
| Copolymère acide méthacrylique / acrylate d'éthyle en émulsion aqueuse à 30% de matière active,commercialisé par la société NOVEON | 2.7 g | 2.7 g |
| Mélange 1-(hexadécyloxy) 2-octadécanol / alcool cétylique | - | 2.5 g |
| Disulfure de sélénium | - | 1 g |
| | | |
| Kaolin | 3 g | - |
| | | |
| Polydiméthylsiloxane de viscosité 500 000 cSt, (Mirasil DM 500 000 de RHODIA) | - | 1.5 g |
| Hydroxyéthylcellulose réticulée à l'épichlorhydrine, quaternisée par la triméthylamine (JR 400 de AMERCHOL) | - | 0.4 g |
| Gomme de guar modifiée par du chlorure de 2,3-époxypropyl triméthyl ammonium (Jaguar C13 S de RHODIA) | 0.1 g | - |
| Hypochlorite de sodium en solution aqueuse à 14% | - | 0.05 g M.A. |
| Acide salicylique | - | 1 g |
| | | |
| Chlorure de sodium | 3 g | - |
| | | |
| Acide citrique | 0.6 g | - |
| | | |
| Conservateurs, parfum | q.s. | q.s. |
| | | |
| Agent de pH q.s.p. | pH 5 | pH 4 |
| | | |
| Eau déminéralisée q.s.p. | 100 g | 100 g |

La composition est stable. Les cheveux mouillés ne sont pas chargés et la mise en forme est aisée.

## Revendications

1. Composition cosmétique, **caractérisée par le fait qu'**elle comprend, dans un milieu cosmétiquement acceptable, au moins un copolymère réticulé d'acide méthacrylique et d'acrylate d'alkyle en C₁-C₄, au moins un polymère cationique ou amphotère et au moins des particules minérales solides insolubles dans l'eau choisies parmi les argiles, les particules essentiellement constituées d'alumine, les particules comprenant au moins 10% en poids de carbonate de calcium et le sulfure de sélénium.

2. Composition selon la revendication 1, **caractérisé en ce que** dans ledit copolymère, l'acide méthacrylique est présent dans des quantités allant de 20 à 80% en poids et plus particulièrement de 25 à 70 % en poids et encore plus particulièrement de 35 à 60% en poids par rapport au poids total du copolymère.

3. Composition selon la revendication 1 ou 2, **caractérisé en ce que** dans ledit copolymère, l'acrylate d'alkyle est présent dans des quantités allant de 15 à 80% en poids et plus particulièrement de 25 à 75 % en poids et encore plus particulièrement de 40 à 65% en poids par rapport au poids total du copolymère.

4. Composition selon l'une quelconque des revendications 1 à 3, **caractérisé en ce que** dans ledit copolymère, l'acrylate d'alkyle est choisi parmi l'acrylate de méthyle, l'acrylate d'éthyle ou l'acrylate de butyle.

5. Composition selon la revendication 4, **caractérisé en ce que** l'acrylate d'alkyle est l'acrylate d'éthyle.

6. Composition selon l'une quelconque des revendications 1 à 5, où le copolymère d'acide méthacrylique et d'acrylate d'alkyle en C₁-C₄ est réticulé par au moins un agent réticulant éthylèniquement polyinsaturé.

7. Composition selon la revendication 6, **caractérisée en ce que** la teneur en agent réticulant varie de 0,01 à 5% en poids et de préférence de 0,03 à 3% en poids et encore plus particulièrement de 0,05 à 1 % en poids par rapport au poids total du copolymère.

8. Compositions selon l'une quelconque des revendications précédentes, **caractérisées par le fait que** l'argile est choisie parmi les argiles de la famille de la kaolinite, de l'halloysite, de la dombassite, de l'antigorite, de la benthiérine, de la pyrophyllite, des montmorillonites, de la beidellite, des vermiculites, du talc, de la stévensite, des hectorites, des saponites, des chlorites, de la sépiolite.

9. Compositions selon la revendication précédente, **caractérisées par le fait que** l'argile est choisie parmi la kaolinite, les montmorillonites, les hectorites et leurs mélanges.

10. Compositions selon l'une quelconque des revendications 1 à 7, **caractérisées par le fait que** la taille primaire moyenne en nombre des particules essentiellement constituées d'alumine est comprise entre 2 et 200 nm, de préférence comprise entre 5 et 50 nm.

11. Composition selon l'une quelconque des revendications précédentes, **caractérisée en ce que** les particules essentiellement constituées d'alumine contiennent au moins 90 % en poids en poids d'alumine.

12. Composition selon l'une quelconque des revendications 1 à 7 et 10 à 11, **caractérisée en ce que** l'alumine est une alumine éventuellement hydratée.

13. Composition selon la revendication précédente, **caractérisée en ce que** l'alumine est la boehmite.

14. Composition selon l'une quelconque des revendications 1 à 7, **caractérisée par le fait que** les particules comprenant au moins 10 % en poids de carbonate de calcium présentent une taille primaire moyenne en nombre comprise entre 2 nm et 2 microns, plus préférentiellement entre 5 et 500 nm, et plus préférentiellement encore entre 10 et 250 nm.

15. Composition selon la revendication précédente, **caractérisée par le fait que** les particules contiennent au moins 50 % en poids de carbonate de calcium, mieux encore au moins 70 % en poids, et encore mieux plus de 90 % en poids de carbonate de calcium.

16. Composition selon l'une quelconque des revendications 14 ou 15, **caractérisée par le fait que** les particules sont des particules de carbonate de calcium substantiellement pur.

17. Composition selon l'une quelconque des revendications 1 à 16, **caractérisée par le fait que** le sulfure de sélénium comprend un atome de sélénium pour deux atomes de soufre ou possède une structure cyclique SexSy dans laquelle x+y=8.

18. Composition selon l'une quelconque des revendications 1 à 7 et 17, **caractérisée en que** le disulfure de sélénium est une poudre dont les particules ont une taille moyenne en nombre inférieure à 200 microns et de préférence inférieure à 25 microns.

19. Composition selon l'une quelconque des revendications 1 à 18, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

20. Composition selon l'une quelconque des revendications précédentes, **caractérisée par le fait que** ledit polymère cationique est choisi parmi :
(1) les homopolymères ou copolymères dérivés d'esters ou d'amides acryliques ou méthacryliques et comportant au moins un des motifs de formules suivantes: 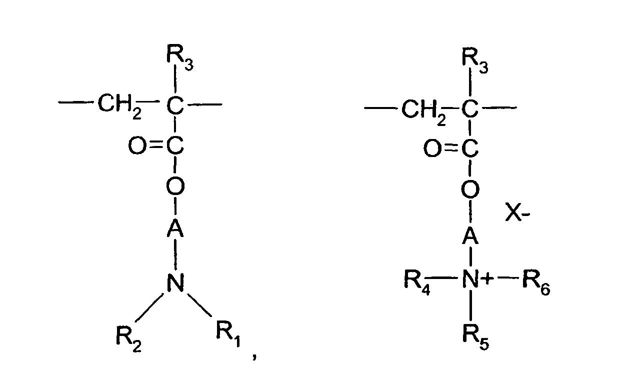 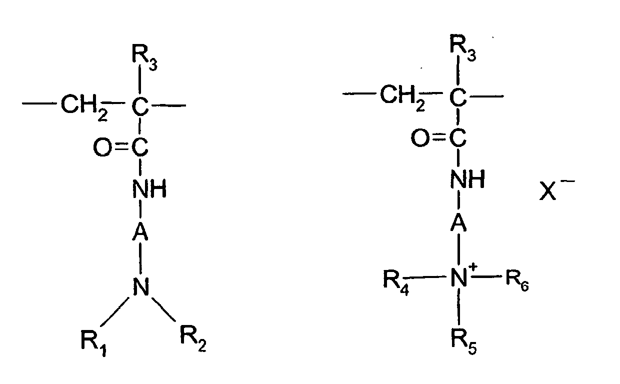 dans lesquelles:
R₃, identiques ou différents, désignent un atome d'hydrogène ou un radical CH₃;
A, identiques ou différents, représentent un groupe alkyle, linéaire ou ramifié, de 1 à 6 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle;
R₁ et R₂ , identiques ou différents, représentent hydrogène ou un groupe alkyle ayant de 1 à 6 atomes de carbone;
X désigne un anion dérivé d'un acide minéral ou organique.
(2) Les polysaccharides cationiques,
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.
(5) les dérivés de polyaminoamides résultant de la condensation de polyalcoylènes polyamines avec des acides polycarboxyliques suivie d'une alcoylation par des agents bifonctionnels,
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
(7) les cyclopolymères d'alkyl diallyl amine ou de dialkyl diallyl ammonium
(8) les polymères de diammonium quaternaire comprenant des motifs récurrents répondant à la formule : 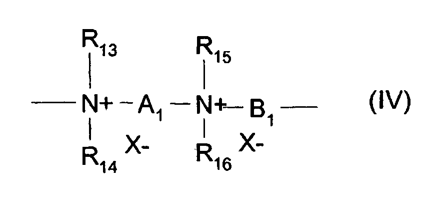 formule (IV) dans laquelle :
R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- ;
De préférence, X⁻ est un anion.
(9) les polymères de polyammonium quaternaires comprenant des motifs de formule (VI): 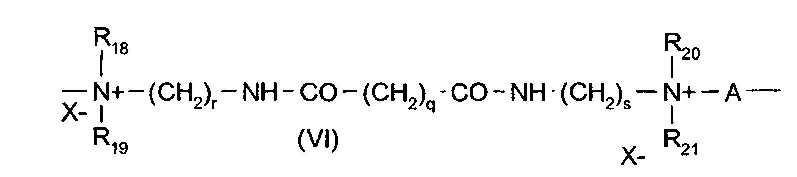 formule dans laquelle :
R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(10) Les polymères quaternaires de vinylpyrrolidone et de vinylimidazole,
(11) Les polyamines référencées sous le nom de « POLYETHYLENEGLYCOL (15) TALLOW POLYAMINE » dans le dictionnaire CTFA.
(12) les polymères réticulés de sels de méthacryloyloxyalkyl(C₁-C₄) trialkyl(C₁-C₄)ammonium,
(13) des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

21. Composition selon la revendication précédente, **caractérisée par le fait que** ledit polymère cationique est choisi parmi les cyclopolymères cationiques, les polysaccharides cationiques, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, les polyalkylèneimines et leurs mélanges.

22. Composition selon la revendication 21, **caractérisée par le fait que** ledit cyclopolymère est choisi parmi les homopolymères du chlorure de diallyldiméthylammonium et les copolymères du chlorure de diallyldiméthylammonium et d'acrylamide.

23. Composition selon la revendication 21, **caractérisée par le fait que** lesdits polysaccharides cationiques sont choisis parmi les gommes de guar modifiées par un sel de 2,3-époxypropyl triméthylammonium et les hydroxyéthylcelluloses ayant réagi avec un époxyde substitué par un groupement triméthylammonium.

24. Composition selon l'une quelconque des revendications 1 à 18, **caractérisée par le fait que** ledit polymère amphotère est choisi parmi :
(1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué comprenant au moins un atome basique tel que plus particulièrement les dialkylaminoalkylméthacrylate et acrylate, les dialkylaminoalkylméthacrylamide et acrylamide. le composé vinylique peut être également un sel de dialkyldiallylammonium tel que le chlorure de diméthyldiallylammonium.
(2) les polymères comportant des motifs dérivant :
a) d'au moins un monomère choisi parmi les acrylamides ou les méthacrylamides substitués sur l'azote par un radical alkyle,
b) d'au moins un comonomère acide comprenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
(3) Les polyaminoamides réticulés et alcoylés partiellement ou totalement dérivant de polyaminoamides de formule générale :
⁅CO-R₄-CO-Z⁆ (VII)
dans laquelle R₄ représente un radical divalent dérivé d'un acide dicarboxylique
saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
a) dans les proportions de 60 à 100 moles %, le radical de formule (VIII) 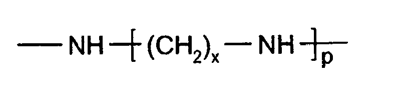 où x=2 et p=2 ou 3, ou bien x=3 et p=2
ce radical dérivant de la diéthylène triamine, de la triéthylène tétraamine ou de la dipropylène triamine;
b) dans les proportions de 0 à 40 moles % le radical (IV) ci-dessus, dans lequel x=2 et p=1 et qui dérive de l'éthylènediamine, ou le radical dérivant de la pipérazine : 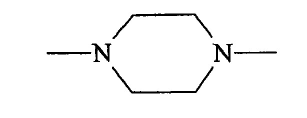
c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
(4) Les polymères comportant des motifs zwittérioniques de formule (IX): 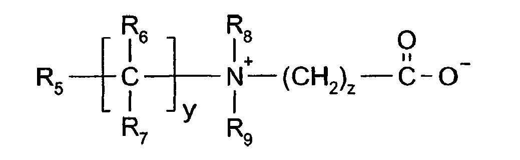 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.
(5) les polymères dérivés du chitosane comportant des motifs monomères répondant aux formules (X), (XI), (XII) suivantes : 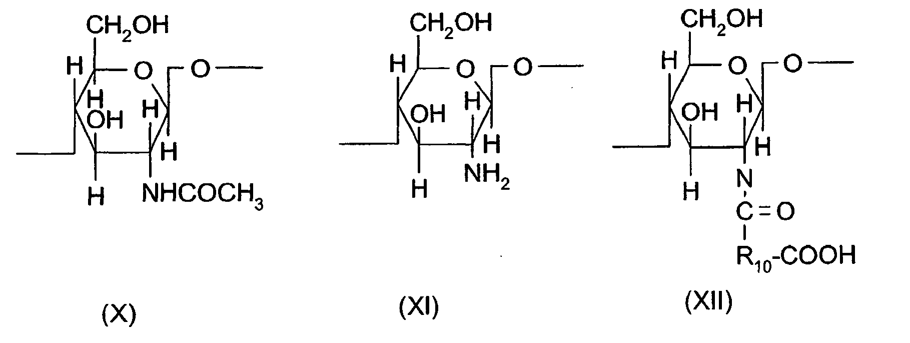 le motif (X) étant présent dans des proportions comprises entre 0 et 30%, le motif (XI) dans des proportions comprises entre 5 et 50% et le motif (XII) dans des proportions comprises entre 30 et 90%, étant entendu que dans ce motif (XII), R₁₀ représente un radical de formule : 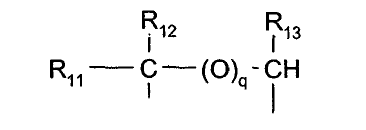 dans laquelle
si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XIII) tels que ceux décrits par exemple dans le brevet français 1 400 366 : 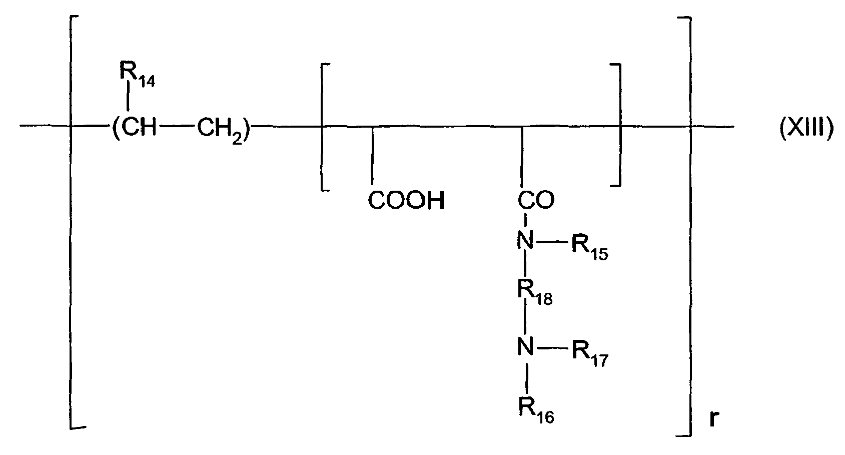 dans laquelle R₁₄ représente un atome d'hydrogène, un radical CH₃O, CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂- , -CH₂-CH₂-CH₂- , -CH₂-CH(CH₃)- , R₁₆ ayant les significations mentionnées ci-dessus,
ainsi que les homologues supérieurs de ces radicaux et comprenant jusqu'à 6 atomes de carbone.
(8) Des polymères amphotères du type -D-X-D-X- choisis parmi:
a) les polymères obtenus par action de l'acide chloracétique ou le chloracétate de sodium sur les composés comportant au moins un motif de formule :
-D-X-D-X-D- (XIV)
où D désigne un radical 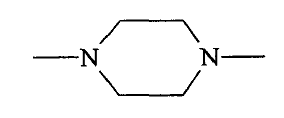 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
b) les polymères de formule :
-D-X-D-X- (XV)
où D désigne un radical 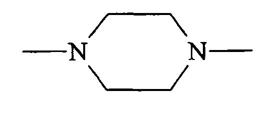
et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine.

25. Composition selon l'une quelconque des revendications précédentes, **caractérisée en ce que** le copolymère acide méthacrylique et d'acrylate d'alkyle en C₁-C₄ est présent à une concentration comprise entre 0,01 % et 20 % en poids par rapport au poids total de la composition, de préférence entre 0,05 % et 15 % en poids.

26. Composition selon l'une quelconque des revendications précédentes, **caractérisée en ce que** les composés minéraux insolubles à une concentration comprise entre 0,001 % et 20 % en poids par rapport au poids total de la composition, de préférence entre 0,01 % et 10 % en poids.

27. Composition selon l'une quelconque des revendications précédentes, **caractérisée en ce que** le polymère cationique ou amphotère est présent à une concentration comprise entre 0,001 % et 20 % en poids par rapport au poids total de la composition, de préférence entre 0,01 % et 10 % en poids.

28. Composition selon l'une quelconque des revendications 1 à 27, **caractérisée par le fait qu'**elle comprend en outre au moins une silicone.

29. Composition selon la revendication 28, **caractérisée par le fait que** les silicones sont des polyorganosiloxanes non volatils choisis parmi les polyalkylsiloxanes, les gommes et résines de silicones, les silicones organomodifiées ainsi que leurs mélanges.

30. Composition selon la revendication 29, **caractérisée par le fait que** :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les gommes de silicone sont choisies parmi les polydiorganosiloxanes ayant des masses molaires moyennes en nombre comprises entre 200 000 et 1 000 000 utilisés seuls ou sous forme de mélange dans un solvant ;
(d) les résines sont choisies parmi les résines comprenant des unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone.

31. Composition selon l'une quelconque des revendications 28 à 30, **caractérisée par le fait que** les silicones additionnelles sont choisies parmi les polyalkylsiloxanes à groupements terminaux triméthylsilyle, les polyalkylsiloxanes à groupements terminaux diméthylsilanol, les mélanges de deux PDMS constitués d'une gomme et d'une huile de viscosités différentes, les mélanges d'organosiloxanes et de silicones cycliques, les résines d'organopolysiloxanes.

32. Composition selon l'une quelconque des revendications 1 à 31, **caractérisée par le fait qu'**elle comprend en outre au moins un agent bénéfique pour les matières kératiniques choisi parmi les esters d'acides carboxyliques en C1-C30 et de d'alcools mono ou polyhydroxylés en C1-C30, les huiles végétales, animales, minérales ou de synthèse, les cires, les céramides, les pseudocéramides.

33. Composition selon l'une quelconque des revendications 28 à 31, **caractérisée en ce que** ladite silicone additionnelle est présente à une concentration comprise entre 0,001 % et 20 % en poids par rapport au poids total de la composition, de préférence entre 0,01 % et 10 % en poids.

34. Composition selon la revendication 32, **caractérisée en ce que** l'agent bénéfique pour les matières kératiniques est présent à une concentration comprise entre 0,001 % et 20 % en poids par rapport au poids total de la composition, de préférence entre 0,01 % et 10 % en poids.

35. Composition selon l'une quelconque des revendications précédentes, **caractérisée par le fait qu'**elle comprend en outre au moins un agent tensioactif choisi parmi les tensioactifs anioniques, non ioniques, amphotères, cationiques et leurs mélanges.

36. Composition selon la revendication 35, **caractérisée par le fait que** le ou les agents tensioactifs sont choisis parmi au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.

37. Composition selon l'une quelconque des revendications 35 ou 36, **caractérisée par le fait que** le ou les agents tensioactifs sont présents à une concentration comprise entre 0,01% et 50% en poids, de préférence entre 0,1% et 40% en poids, et encore plus préférentiellement entre 0,5% et 30% en poids, par rapport au poids total de la composition.

38. Composition selon l'une quelconques des revendications précédentes, **caractérisée en qu'**elle comprend de 3 à 50% en poids d'un tensioactif détergent.

39. Composition selon l'une quelconque des revendications 1 à 38, **caractérisée par** le fait la composition contient au moins un additif choisi parmi les épaississants, les agents antipelliculaires ou anti-séborrhéiques, les parfums, les agents nacrants, les hydroxyacides, les électrolytes, les esters d'acides gras, les conservateurs, les filtres solaires siliconés ou non, les vitamines, les provitamines telles que le panthénol, les polymères anioniques ou non ioniques, les protéines, les hydrolysats de protéines, l'acide méthyl-18 eicosanoique, les huiles fluorées ou perfluorées, les amines grasses, les acides gras et leurs dérivés, les alcools gras et leurs dérivés ainsi que les mélanges de ces différents composés.

40. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

41. Utilisation d'une composition telle que définie dans l'une quelconque des revendications précédentes pour le lavage ou pour le soin des matières kératiniques.

42. Procédé de traitement des matières kératiniques, telles que les cheveux, **caractérisé en ce qu'**il consiste à appliquer sur lesdites matières une composition cosmétique selon l'une quelconque des revendications 1 à 40, puis à effectuer éventuellement un rinçage à l'eau.

43. Utilisation d'un copolymère réticulé acide méthacrylique / acrylate d'alkyle en C₁-C₄ dans, ou pour la fabrication d'une composition cosmétique comprenant un polymère cationique ou amphotère et au moins des particules minérales solides insolubles dans l'eau choisies parmi les argiles, les particules essentiellement constituées d'alumine, les particules comprenant au moins 10 % en poids de carbonate de calcium et le sulfure de sélénium.

44. Utilisation d'une composition telle que définie dans l'une quelconque des revendications 1 à 40 pour apporter de la souplesse aux cheveux.

## Claims

1. Cosmetic composition, **characterized in that** it comprises, in a cosmetically acceptable medium, at least one crosslinked copolymer of methacrylic acid and of a C₁-C₄ alkyl acrylate, at least one cationic or amphoteric polymer and at least water-insoluble solid mineral particles chosen from clays, particles consisting essentially of alumina, particles comprising at least 10% by weight of calcium carbonate, and selenium sulfide.

2. Composition according to Claim 1, **characterized in that,** in the said copolymer, the methacrylic acid is present in amounts ranging from 20% to 80% by weight, more particularly from 25% to 70% by weight and even more particularly from 35% to 60% by weight relative to the total weight of the copolymer.

3. Composition according to Claim 1 or 2, **characterized in that,** in the said copolymer, the alkyl acrylate is present in amounts ranging from 15% to 80% by weight, more particularly from 25% to 75% by weight and even more particularly from 40% to 65% by weight relative to the total weight of the copolymer.

4. Composition according to any one of Claims 1 to 3, **characterized in that,** in the said copolymer, the alkyl acrylate is chosen from methyl acrylate, ethyl acrylate and butyl acrylate.

5. Composition according to Claim 4, **characterized in that** the alkyl acrylate is ethyl acrylate.

6. Composition according to any one of Claims 1 to 5, in which the copolymer of methacrylic acid and of a C₁-C₄ alkyl acrylate is crosslinked with at least one polyethylenically unsaturated crosslinking agent.

7. Composition according to Claim 6, **characterized in that** the content of crosslinking agent ranges from 0.01% to 5% by weight, preferably from 0.03% to 3% by weight and even more particularly from 0.05% to 1% by weight relative to the total weight of the copolymer.

8. Composition according to any one of the preceding claims, **characterized in that** the clay is chosen from clays of the kaolinite, halloysite, dombassite, antigorite, benthierine, pyrophyllite, montmorillonite, beidellite, vermiculite, talc, stevensite, hectorite, saponite, chlorite or sepiolite family.

9. Composition according to the preceding claim, **characterized in that** the clay is chosen from kaolinite, montmorillonites and hectorites, and mixtures thereof.

10. Composition according to any one of Claims 1 to 7, **characterized in that** the number-average primary size of the particles consisting essentially of alumina is between 2 and 200 nm and preferably between 5 and 50 nm.

11. Composition according to any one of the preceding claims, **characterized in that** the particles consisting essentially of alumina contain at least 90% by weight of alumina.

12. Composition according to any one of Claims 1 to 7, 10 and 11, **characterized in that** the alumina is an optionally hydrated alumina.

13. Composition according to the preceding claim, **characterized in that** the alumina is boehmite.

14. Composition according to any one of Claims 1 to 7, **characterized in that** the particles comprising at least 10% by weight of calcium carbonate have a number-average primary size of between 2 nm and 2 microns, more preferably between 5 and 500 nm and even more preferably between 10 and 250 nm.

15. Composition according to the preceding claim, **characterized in that** the particles contain at least 50% by weight of calcium carbonate, better still at least 70% by weight and even better still more than 90% by weight of calcium carbonate.

16. Composition according to either of Claims 14 and 15, **characterized in that** the particles are particles of substantially pure calcium carbonate.

17. Composition according to any one of Claims 1 to 16, **characterized in that** the selenium sulfide comprises one selenium atom per two sulfur atoms or has a SexSy cyclic structure in which x+y=8.

18. Composition according to any one of Claims 1 to 7 and 17, **characterized in that** the selenium disulfide is a powder with a number-average particle size of less than 200 microns and preferably less than 25 microns.

19. Composition according to any one of Claims 1 to 18, **characterized in that** the cationic polymers are chosen from those containing units comprising primary, secondary, tertiary and/or quaternary amine groups that may either form part of the main polymer chain or may be borne by a side substituent directly attached thereto.

20. Composition according to any one of the preceding claims, **characterized in that** the said cationic polymer is chosen from:
(1) homopolymers or copolymers derived from acrylic or methacrylic esters or amides comprising at least one of the units having the following formulae: 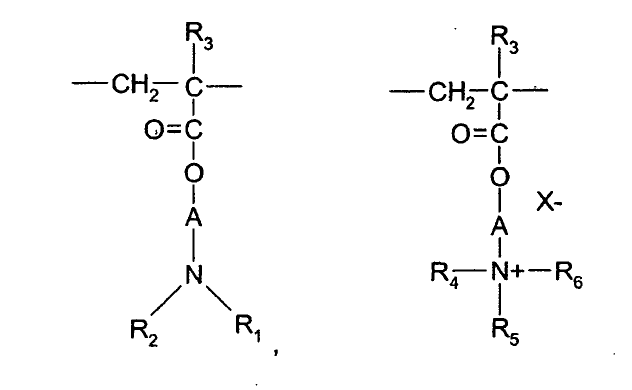 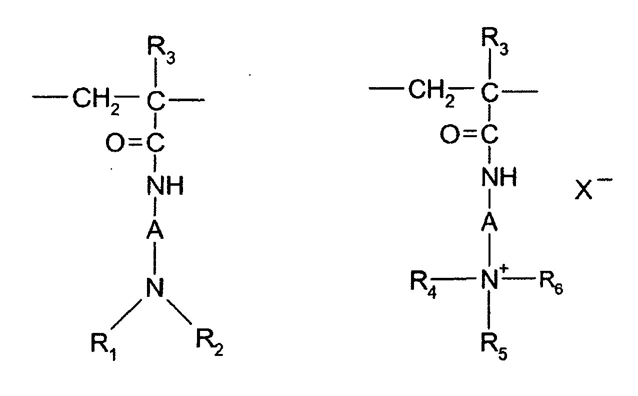 in which:
R₃, which may be identical or different, denote a hydrogen atom or a CH₃ radical;
A, which may be identical or different, represent a linear or branched alkyl group of 1 to 6 carbon atoms or a hydroxyalkyl group of 1 to 4 carbon atoms;
R₄, R₅ and R₆, which may be identical or different, represent an alkyl group containing from 1 to 18 carbon atoms or a benzyl radical;
R₁ and R₂, which may be identical or different, represent hydrogen or an alkyl group containing from 1 to 6 carbon atoms;
X denotes an anion derived from a mineral or organic acid;
(2) cationic polysaccharides;
(3) polymers constituted of piperazinyl units and of divalent alkylene or hydroxyalkylene radicals containing straight or branched chains, optionally interrupted by oxygen, sulfur or nitrogen atoms or by aromatic or heterocyclic rings, as well as the oxidation and/or quaternization products of these polymers;
(4) water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized;
(5) polyamino amide derivatives resulting from the condensation of polyalkylene polyamines with polycarboxylic acids followed by alkylation with difunctional agents;
(6) polymers obtained by reaction of a pclyalkylene polyamine containing two primary amine groups and at least one secondary amine group with a dicarboxylic acid chosen from diglycolic acid and saturated aliphatic dicarboxylic acids having from 3 to 8 carbon atoms;
(7) cyclopolymers of alkyldiallylamine or of dialkyldiallylammonium;
(8) quaternary diammonium polymers containing repeating units corresponding to the formula: 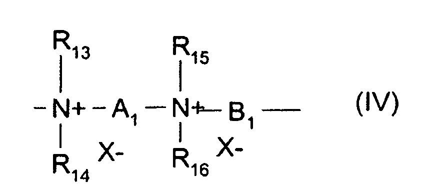 in which formula (IV):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second heteroatom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms, which groups may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
X⁻ denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon-based radical or a group corresponding to one of the following formulae:
-(CH₂-CH₂-O)ₓ-CH₂-CH₂-
- [C_{H}2-CH(CH₃)-O]_{y}-CH₂-CH(CH₃)-
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon-based radical, or alternatively the divalent radical
-CH₂-CH₂-S-S-CH₂-CH₂-;
d) a ureylene group of formula: -NH-CO-NH-;
preferably, X⁻ is an anion;
(9) polyquaternary ammonium polymers consisting of units of formula (VI): 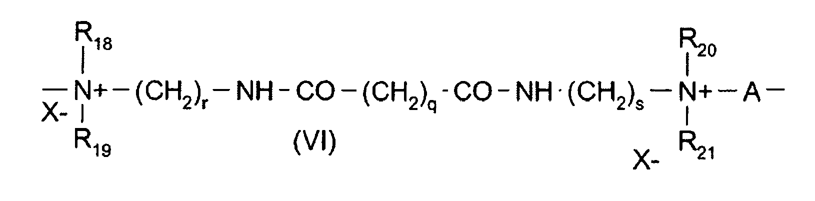 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂-;
(10) quaternary polymers of vinylpyrrolidone and of vinylimidazole;
(11) the polyamines referenced under the name "Polyethylene Glycol (15) Tallow Polyamine" in the CTFA dictionary;
(12) crosslinked polymers of methacryloyloxy(C₁-C₄)alkyltri(C₁-C₄)alkylammonium salts;
(13) polyalkyleneimines, in particular polyethyleneimines, polymers comprising vinylpyridine or vinylpyridinium units, condensates of polyamines and of epichlorohydrin, quaternary polyureylenes, and chitin derivatives.

21. Composition according to the preceding claim, **characterized in that** the said cationic polymer is chosen from cationic cyclopolymers, cationic polysaccharides, quaternary polymers of vinylpyrrolidone and of vinylimidazole, crosslinked homopolymers or copolymers of methacryloyloxy-(C₁-C₄)alkyltri(C₁-C₄)alkylammonium salts, and polyalkyleneimines, and mixtures thereof.

22. Composition according to Claim 21, **characterized in that** the said cyclopolymer is chosen from diallyldimethylammonium chloride homopolymers and copolymers of diallyldimethylammonium chloride and of acrylamide.

23. Composition according to Claim 21, **characterized in that** the said cationic polysaccharides are chosen from guar gums modified with a 2,3-epoxypropyltrimethylammonium salt and hydroxyethylcelluloses that have reacted with an epoxide substituted with a trimethylammonium group.

24. Composition according to any one of Claims 1 to 18, **characterized in that** the said amphoteric polymer is chosen from:
(1) polymers resulting from the copolymerization of a monomer derived from a vinyl compound bearing a carboxylic group such as, more particularly, acrylic acid, methacrylic acid, maleic acid, α-chloroacrylic acid, and a basic monomer derived from a substituted vinyl compound containing at least one basic atom, such as, more particularly, dialkylaminoalkyl methacrylate and acrylate, dialkylaminoalkylmethacrylamide and -acrylamide; the vinyl compound may also be a dialkyldiallylammonium salt such as dimethyldiallylammonium chloride;
(2) polymers containing units derived from:
a) at least one monomer chosen from acrylamides and methacrylamides substituted on the nitrogen with an alkyl radical,
b) at least one acidic comonomer containing one or more reactive carboxylic groups, and
c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate;
(3) crosslinked and partially or totally alkylated polyamino amides derived from polyamino amides of general formula:
⁅CO-R₄-CO-Z⁆ (VII)
in which R₄ represents a divalent radical derived from a saturated dicarboxylic acid, a mono- or dicarboxylic aliphatic acid containing an ethylenic double bond, an ester of a lower alkanol, having 1 to 6 carbon atoms of these acids or a radical derived from the addition of any one of the said acids to a bis(primary) or bis(secondary) amine, and Z denotes a bis(primary), mono- or bis(secondary) polyalkylene-polyamine radical and preferably represents:
a) in proportions of from 60 to 100 mol%, the radical of formula (VIII) 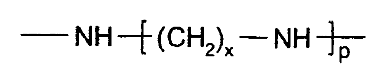 where x = 2 and p = 2 or 3, or alternatively x = 3 and p = 2
this radical being derived from diethylenetriamine, from triethylenetetraamine or from dipropylenetriamine;
b) in proportions of from 0 to 40 mol%, the radical (IV) above in which x = 2 and p = 1 and which is derived from ethylenediamine, or the radical derived from piperazine: 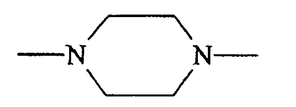
c) in proportions of from 0 to 20 mol%, the -NH-(CH₂)₆-NH- radical derived from hexamethylenediamine, these polyamino amines being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof;
(4) polymers comprising zwitterionic units of formula (IX): 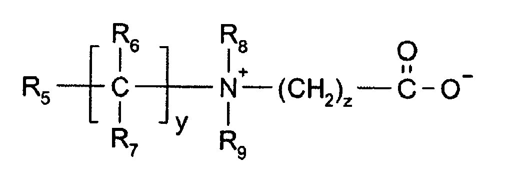 in which R₅ denotes a polymerizable unsaturated group such as an acrylate, methacrylate, acrylamide or methacrylamide group, y and z represent an integer from 1 to 3, R₆ and R₇ represent a hydrogen atom, methyl, ethyl or propyl, R₈ and R₉ represent a hydrogen atom or an alkyl radical such that the sum of the carbon atoms in R₈ and R₉ does not exceed 10;
(5) polymers derived from chitosan comprising monomer units corresponding to formulae (X), (XI) and (XII) below: 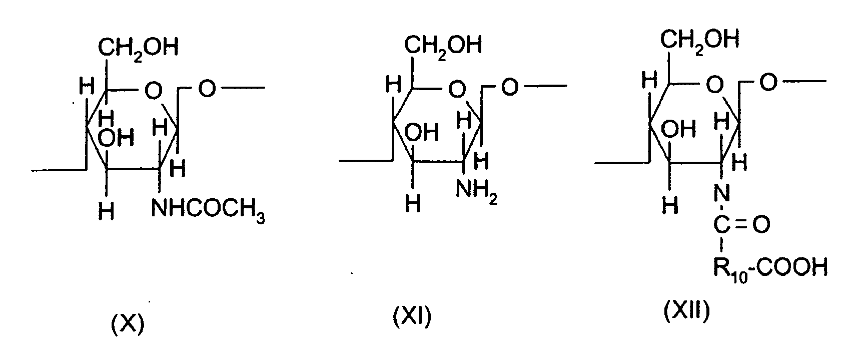 the unit (X) being present in proportions of between 0 and 30%, the unit (XI) in proportions of between 5 and 50% and the unit (XII) in proportions of between 30 and 90%, it being understood that, in this unit (XII), R₁₀ represents a radical of formula: 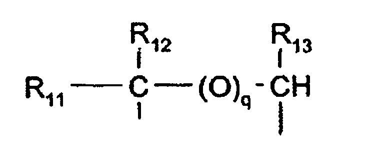 in which
if q = 0, R₁₁, R₁₂ and R₁₃, which may be identical or different, each represent a hydrogen atom, a methyl, hydroxyl, acetoxy or amino residue, a monoalkylamine residue or a dialkylamine residue which are optionally interrupted by one or more nitrogen atoms and/or optionally substituted with one or more amine, hydroxyl, carboxyl, alkylthio or sulfonic groups, an alkylthio residue in which the alkyl group bears an amino residue, at least one of the radicals R₁₁, R₁₂ and R₁₃ being, in this case, a hydrogen atom;
or, if q = 1, R₁₁, R₁₂ and R₁₃ each represent a hydrogen atom, as well as the salts formed by these compounds with bases or acids;
(6) polymers derived from the N-carboxyalkylation of chitosan, such as N-carboxymethylchitosan or N-carboxybutylchitosan;
(7) polymers corresponding to the general formula (XIII) as described, for example, in French patent 1 400 366: 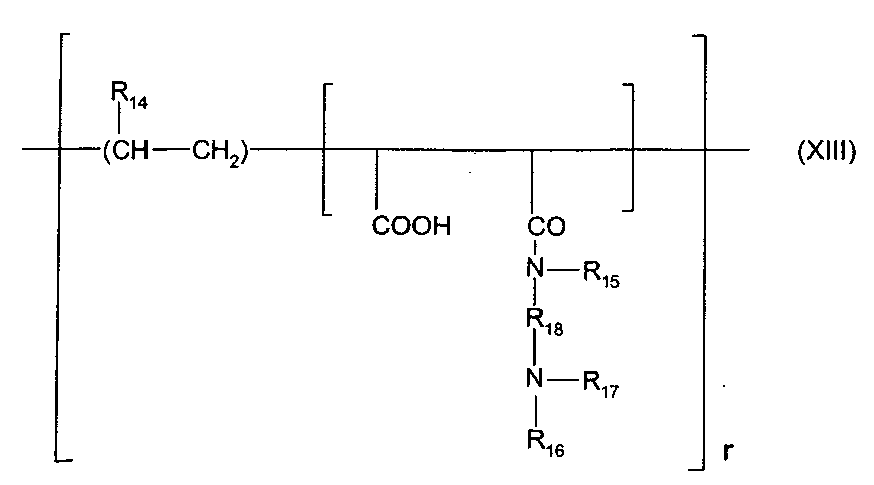 in which R₁₄ represents a hydrogen atom, a CH₃O, CH₃CH₂O or phenyl radical, R₁₅ denotes hydrogen or a lower alkyl radical such as methyl or ethyl, R₁₆ denotes hydrogen or a lower alkyl radical such as methyl or ethyl, R₁₇ denotes a lower alkyl radical such as methyl or ethyl or a radical corresponding to the formula: -R₁₈-N(R₁₆)₂, R₁₈ representing a -CH₂-CH₂-, -CH₂-CH₂-CH₂- or -CH₂-CH(CH₃)- group, R₁₆ having the meanings mentioned above,
as well as the higher homologues of these radicals and containing up to 6 carbon atoms;
(8) amphoteric polymers of the type -D-X-D-X-chosen from:
a) polymers obtained by the action of chloroacetic acid or sodium chloroacetate on compounds containing at least one unit of formula:
-D-X-D-X-D- (XIV)
where D denotes a radical 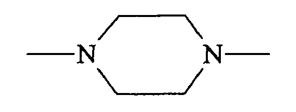 and X denotes the symbol E or E', E or E', which may be identical or different, denoting a divalent radical which is an alkylene radical with a straight or branched chain containing up to 7 carbon atoms in the main chain, which is unsubstituted or substituted with hydroxyl groups and which can contain, in addition to the oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;
b) polymers of formula:
-D-X-D-X- (XV)
where D denotes a radical 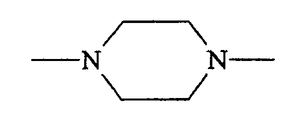 and X denotes the symbol E or E' and at least once E'; E having the meaning given above and E' being a divalent radical which is an alkylene radical with a straight or branched chain having up to 7 carbon atoms in the main chain, which is unsubstituted or substituted with one or more hydroxyl radicals and containing one or more nitrogen atoms, the nitrogen atom being substituted with an alkyl chain which is optionally interrupted by an oxygen atom and necessarily containing one or more carboxyl functions or one or more hydroxyl functions and betainized by reaction with chloroacetic acid or sodium chloroacetate;
(9) (C₁-C₅)alkyl vinyl ether/maleic anhydride copolymers partially modified by semiamidation with an N,N-dialkylaminoalkylamine such as N,N-dimethylaminopropylamine or by semiesterification with an N,N-dialkanolamine.

25. Composition according to any one of the preceding claims, **characterized in that** the copolymer of methacrylic acid and of a C₁-C₄ alkyl acrylate is present in a concentration of between 0.01% and 20% by weight and preferably between 0.05% and 15% by weight relative to the total weight of the composition.

26. Composition according to any one of the preceding claims, **characterized in that** the insoluble mineral compounds are present in a concentration of between 0.001% and 20% by weight and preferably between 0.01% and 10% by weight relative to the total weight of the composition.

27. Composition according to any one of the preceding claims, **characterized in that** the cationic or amphoteric polymer is present in a concentration of between 0.001% and 20% by weight and preferably between 0.01% and 10% by weight relative to the total weight of the composition.

28. Composition according to any one of Claims 1 to 27, **characterized in that** it also comprises at least one silicone.

29. Composition according to Claim 28, **characterized in that** the silicones are non-volatile polyorganosiloxanes chosen from polyalkylsiloxanes, silicone gums and resins, and organomodified silicones, and mixtures thereof.

30. Composition according to Claim 29, **characterized in that:**
(a) the polyalkylsiloxanes are chosen from:
- polydimethylsiloxanes containing trimethylsilyl end groups;
- polydimethylsiloxanes containing dimethylsilanol end groups;
- poly(C₁-C₂₀)alkylsiloxanes;
(b) the silicone gums are chosen from polydiorganosiloxanes with number-average molar masses of between 200 000 and 1 000 000, used alone or in the form of a mixture in a solvent;
(d) the resins are chosen from resins comprising units: R₃SiO_{1/2}, R₂SiO_{2/2}, RSiO_{3/2}, SiO_{4/2}
in which R represents a hydrocarbon-based group containing from 1 to 16 carbon atoms.

31. Composition according to any one of Claims 28 to 30, **characterized in that** the additional silicones are chosen from polyalkylsiloxanes containing trimethylsilyl end groups, polyalkylsiloxanes containing dimethylsilanol end groups, mixtures of two PDMSs consisting of a gum and an oil with different viscosities, mixtures of organosiloxanes and of cyclic silicones, and organopolysiloxane resins.

32. Composition according to any one of Claims 1 to 31, **characterized in that** it also comprises at least one agent that is beneficial to keratin materials, chosen from esters of C₁-C₃₀ carboxylic acids and of C₁-C₃₀ monohydroxylated or polyhydroxylated alcohols, plant, animal, mineral or synthetic oils, waxes, ceramides and pseudoceramides.

33. Composition according to any one of Claims 28 to 31, **characterized in that** the said additional silicone is present in a concentration of between 0.001% and 20% by weight and preferably between 0.01% and 10% by weight relative to the total weight of the composition.

34. Composition according to Claim 32, **characterized in that** the agent that is beneficial to keratin materials is present in a concentration of between 0.001% and 20% by weight and preferably between 0.01% and 10% by weight relative to the total weight of the composition.

35. Composition according to any one of the preceding claims, **characterized in that** it also comprises at least one surfactant chosen from anionic, nonionic, amphoteric and cationic surfactants, and mixtures thereof.

36. Composition according to Claim 35, **characterized in that** the surfactant(s) is (are) chosen from at least one or more anionic surfactants or mixtures of at least one or more anionic surfactants and of at least one or more amphoteric surfactants or of at least one or more nonionic surfactants.

37. Composition according to either of Claims 35 and 36, **characterized in that** the surfactant(s) is (are) present in a concentration of between 0.01% and 50% by weight, preferably between 0.1% and 40% by weight and even more preferably between 0.5% and 30% by weight, relative to the total weight of the composition.

38. Composition according to any one of the preceding claims, **characterized in that** it comprises from 3% to 50% by weight of a detergent surfactant.

39. Composition according to any one of Claims 1 to 38, **characterized in that** the composition contains at least one additive chosen from thickeners, antidandruff agents, anti-seborrhoeic agents, fragrances, nacres, hydroxy acids, electrolytes, fatty acid esters, preserving agents, silicone or non-silicone sunscreens, vitamins, provitamins such as panthenol, anionic or nonionic polymers, proteins, protein hydrolysates, 18-methyleicosanoic acid, fluoro or perfluoro oils, fatty amines, fatty acids and derivatives thereof, fatty alcohols and derivatives thereof, and also mixtures of these various compounds.

40. Composition according to any one of the preceding claims, **characterized in that** it is in the form of a shampoo, a conditioner, a composition for permanent-waving, relaxing, dyeing or bleaching the hair, a rinse-out composition to be applied between the two steps of a permanent-waving or hair-relaxing operation, or a washing composition for the body.

41. Use of a composition as defined in any one of the preceding claims, for washing or caring for keratin materials.

42. Process of treating keratin materials, such as the hair, **characterized in that** it consists in applying to the said materials a cosmetic composition according to any one of Claims 1 to 40, and then in optionally rinsing with water.

43. Use of a methacrylic acid/C₁-C₄ alkyl acrylate crosslinked copolymer in, or for the manufacture of, a cosmetic composition comprising a cationic or amphoteric polymer and at least water-insoluble solid mineral particles chosen from clays, particles consisting essentially of alumina, particles comprising at least 10% by weight of calcium carbonate, and selenium sulfide.

44. Use of a composition as defined in any one of Claims 1 to 40, to give the hair suppleness.

## Patentansprüche

1. Kosmetische Zusammensetzung, **dadurch gekennzeichnet, dass** sie in einem kosmetisch akzeptablen Medium mindestens ein vernetztes Copolymer von Methacrylsäure und Alkyl(C₁₋₄)acrylat, mindestens ein kationisches oder amphoteres Polymer, zumindest in Wasser unlösliche feste Mineralpartikel, die unter den Tonen ausgewählt sind, Partikel, die im Wesentlichen aus Aluminiumoxid bestehen, Partikel, die mindestens 10 Gew.-% Calciumcarbonat enthalten, und Selensulfid enthält.

2. Zusammensetzung nach Anspruch 1, **dadurch gekennzeichnet, dass** die Methacrylsäure in dem Copolymer in Mengenanteilen von 20 bis 80 Gew.-%, insbesondere 25 bis 70 Gew.-% und besonders 35 bis 60 Gew.-%, bezogen auf das Gesamtgewicht des Copolymers, vorliegt.

3. Zusammensetzung nach Anspruch 1 oder 2, **dadurch gekennzeichnet, dass** das Alkylacrylat in dem Copolymer in Mengenanteilen von 15 bis 80 Gew.-%, insbesondere 25 bis 75 Gew.-% und besonders 40 bis 65 Gew.-%, bezogen auf das Gesamtgewicht des Copolymers, vorliegt.

4. Zusammensetzung nach einem der Ansprüche 1 bis 3, **dadurch gekennzeichnet, dass** das Alkylacrylat in dem Copolymer unter Methylacrylat, Ethylacrylat oder Butylacrylat ausgewählt ist.

5. Zusammensetzung nach Anspruch 4, **dadurch gekennzeichnet, dass** es sich bei dem Alkylacrylat um das Ethylacrylat handelt.

6. Zusammensetzung nach einem der Ansprüche 1 bis 5, wobei das Copolymer von Methacrylsäure und Alkyl(C₁₋₄)acrylat mit mindestens einem mehrfach ethylenisch ungesättigten Vernetzungsmittel vernetzt ist.

7. Zusammensetzung nach Anspruch 6, **dadurch gekennzeichnet, dass** der Gehalt des Vernetzungsmittels im Bereich von 0,01 bis 5 Gew.-% und vorzugsweise 0,03 bis 3 Gew.-% und insbesondere 0,05 bis 1 Gew.-%, bezogen auf das Gesamtgewicht des Copolymers, liegt.

8. Zusammensetzung nach einem der vorhergehenden Ansprüche, **dadurch gekennzeichnet, dass** der Ton unter den Tonen der Kaolinit-Gruppe, den Tonen der Halloysit-Gruppe, den Tonen der Donbassit-Gruppe, den Tonen der Antigorit-Gruppe, den Tonen der Berthierin-Gruppe, den Tonen der Pyrophyllit-Gruppe, den Montmorilloniten, den Tonen der Beidellit-Gruppen, den Vermiculiten, den Tonen der Talkgruppe, den Tonen der Stevensit-Gruppe, den Hectoriten, den Saponiten, den Chloriten und den Tonen der Sepiolith-Gruppe ausgewählt ist.

9. Zusammensetzung nach dem vorhergehenden Anspruch, **dadurch gekennzeichnet, dass** der Ton unter Kaolinit, den Montmorilloniten, den Hectoriten und deren Gemischen ausgewählt ist.

10. Zusammensetzung nach einem der Ansprüche 1 bis 7, **dadurch gekennzeichnet, dass** die zahlenmittlere Primärgröße der Partikel, die im Wesentlichen aus Aluminiumoxid bestehen, im Bereich von 2 bis 200 nm und vorzugsweise im Bereich von 5 bis 50 nm liegt.

11. Zusammensetzung nach einem der vorhergehenden Ansprüche, **dadurch gekennzeichnet, dass** die Partikel, die im Wesentlichen aus Aluminiumoxid bestehen, zumindest 90 Gew.-% Aluminiumoxid enthalten.

12. Zusammensetzung nach einem der Ansprüche 1 bis 7 und 10 bis 11, **dadurch gekennzeichnet, dass** es sich bei dem Aluminiumoxid um ein gegebenenfalls hydratisiertes Aluminiumoxid handelt.

13. Zusammensetzung nach dem vorhergehenden Anspruch, **dadurch gekennzeichnet, dass** es sich bei dem Aluminiumoxid um Böhmit handelt.

14. Zusammensetzung nach einem der Ansprüche 1 bis 7, **dadurch gekennzeichnet, dass** die Partikel, die mindestens 10 Gew.-% Calciumcarbonat enthalten, eine zahlenmittlere Primärgröße im Bereich von 2 nm bis 2 µm, vorzugsweise im Bereich von 5 bis 500 nm und noch bevorzugter im Bereich von 10 bis 250 nm aufweisen.

15. Zusammensetzung nach dem vorhergehenden Anspruch, **dadurch gekennzeichnet, dass** die Partikel mindestens 50 Gew.-% Calciumcarbonat, besser mindestens 70 Gew.-% Calciumcarbonat und noch besser mehr als 90 Gew.-% Calciumcarbonat enthalten.

16. Zusammensetzung nach einem der Ansprüche 14 oder 15, **dadurch gekennzeichnet, dass** es sich bei den Partikeln um Partikel aus im Wesentlichen reinem Calciumcarbonat handelt.

17. Zusammensetzung nach einem der Ansprüche 1 bis 16, **dadurch gekennzeichnet, dass** das Selensulfid ein Selenatom auf zwei Schwefelatome aufweist oder eine cyclische Struktur SexSy mit x+y=8 besitzt.

18. Zusammensetzung nach einem der Ansprüche 1 bis 7 und 17, **dadurch gekennzeichnet, dass** das Selendisulfid ein Pulver ist, dessen Partikel eine zahlenmittlere Größe unter 200 µm und vorzugsweise unter 25 µm aufweisen.

19. Zusammensetzung nach einem der Ansprüche 1 bis 18, **dadurch gekennzeichnet, dass** die kationischen Polymere unter den Polymeren ausgewählt sind, die Einheiten enthalten, die primäre, sekundäre, tertiäre und/oder quartäre Aminogruppen aufweisen, die entweder Teil der Polymerhauptkette sind oder von einem direkt an diese gebundenen seitlichen Substituenten getragen werden.

20. Zusammensetzung nach einem der vorhergehenden Ansprüche, **dadurch gekennzeichnet, dass** das kationische Polymer ausgewählt ist unter:
(1) Homopolymeren oder Copolymeren, die von Acrylsäureestern, Methacrylsäureestern, Acrylamiden oder Methacrylamiden abgeleitet sind und mindestens eine der Einheiten der folgenden Formeln enthalten: 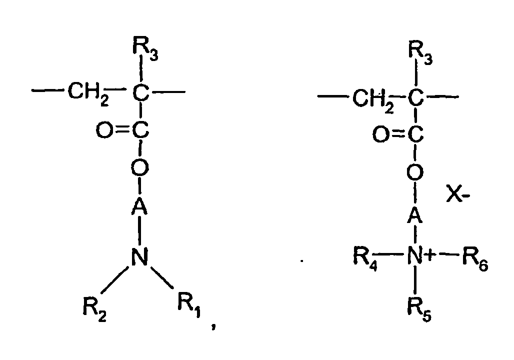 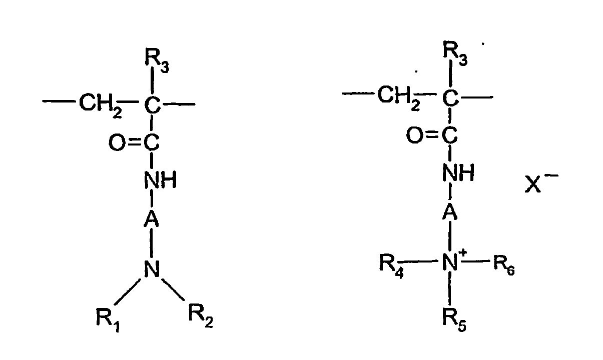 in den Formeln:
die Gruppen R₃, die gleich oder verschieden sind, bedeuten ein Wasserstoffatom oder die Gruppe CH₃;
die Gruppen A, die gleich oder verschieden sind, bedeuten eine lineare oder verzweigte Alkylgruppe mit 1 bis 6 Kohlenstoffatomen oder eine Hydroxyalkylgruppe mit 1 bis 4 Kohlenstoffatomen;
die Gruppen R₄, R₅ und R₆, die gleich oder verschieden sind, bedeuten eine Alkylgruppe mit 1 bis 18 Kohlenstoffatomen oder eine Benzylgruppe;
die Gruppen R₁ und R₂, die gleich oder verschieden sind, bedeuten Wasserstoff oder eine Alkylgruppe mit 1 bis 6 Kohlenstoffatomen;
die Gruppe X bedeutet ein Anion, das von einer anorganischen oder organischen Säure abgeleitet ist;
(2) kationischen Polysacchariden;
(3) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder durch aromatische oder heterocyclische Ringe unterbrochen sind, sowie den Oxidationsprodukten und/oder Quaternisierungsprodukten dieser Polymere;
(4) in Wasser löslichen Polyaminoamiden, die insbesondere durch Polykondensation einer Säure mit einem Polyamin hergestellt werden; wobei dieser Polyaminoamide mit einem Epihalohydrin, Diepoxid, Dianhydrid, ungesättigtem Dianhydrid, zweifach ungesättigtem Derivat, Bis-halohydrin, Bis-azetidinium, Bis-haloacyldiamin, Alkyl-bis-halogenid oder auch einem Oligomer vernetzt sein können, das bei der Reaktion einer bifunktionellen Verbindung gebildet wird, die gegenüber einem Bis-halohydrin, Bis-azetidinium, Bis-haloacyldiamin, Alkyl-bis-halogenid, Epihalohydrin, Diepoxid oder einem zweifach ungesättigten Derivat reaktiv ist; wobei das Vernetzungsmittel in Mengenanteilen von 0,025 bis 0,35 mol pro Aminogruppe des Polyaminoamids verwendet wird; diese Polyaminoamide können alkyliert sein oder, wenn sie eine oder mehrere tertiäre Aminofunktionen besitzen, quaternisiert sein;
(5) Polyaminoamidderivaten, die bei der Kondensation von Polyalkylenpolyaminen mit Polycarbonsäuren und anschließender Alkylierung mit bifunktionellen Stoffen gebildet werden;
(6) Polymeren, die durch Umsetzung eines Polyalkylenpolyamins mit zwei primären Aminogruppen und mindestens einer sekundären Aminogruppe und einer Dicarbonsäure gebildet werden, die unter Diglycolsäure und den gesättigten aliphatischen Dicarbonsäuren mit 3 bis 8 Kohlenstoffatomen ausgewählt ist;
(7) Alkyldiallylamin-Cyclopolymeren oder Dialkyldiallylammonium-Cyclopolymeren;
(8) Quartären Diammoniumpolymeren, die Wiederholungseinheiten der folgenden Formel enthalten: 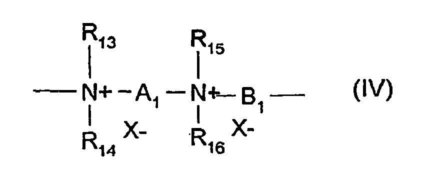 in der Formel (IV):
die Gruppen R₁₃, R₁₄, R₁₅ und R₁₆, die identisch oder voneinander verschieden sind, bedeuten aliphatische, alicyclische oder arylaliphatische Gruppen mit 1 bis 20 Kohlenstoffatomen oder niedere hydroxyalkylaliphatische Gruppen oder die Gruppen R₁₃, R₁₄, R₁₅ und R₁₆ bilden gemeinsam oder getrennt voneinander mit den Stickstoffatomen, an die sie gebunden sind, Heterocyclen, die gegebenenfalls ein von Stickstoff verschiedenes, zweites Heteroatom enthalten, oder die Gruppen R₁₃, R₁₄, R₁₅ und R₁₆ bedeuten eine geradkettige oder verzweigte C₁₋₆-Alkylgruppe, die mit einer Nitrilgruppe, Estergruppe, Acylgruppe, Amidgruppe oder -CO-O-R₁₇-D oder -CO-NH-R₁₇-D substituiert ist, wobei R₁₇ eine Alkylengruppe bedeutet und D eine quartäre Ammoniumgruppe ist;
die Gruppen A₁ und B₁ bedeuten Polymethylengruppen, die 2 bis 20 Kohlenstoffatome aufweisen und linear oder verzweigt, gesättigt oder ungesättigt sein können und an die Hauptkette gebunden oder eingebaut in die Hauptkette einen oder mehrere aromatische Ringe oder ein oder mehrere Sauerstoffatome, Schwefelatome oder eine oder mehrere der folgenden Gruppen enthalten können: Sulfoxid, Sulfon, Disulfid, Amino, Alkylamino, Hydroxy, quartäres Ammonium, Ureido, Amid oder Ester, und
X⁻ bedeutet ein Anion, das von einer anorganischen oder organischen Säure abgeleitet ist;
A₁, R₁₃ und R₁₅ können mit den beiden Stickstoffatomen, an die sie gebunden sind, einen Piperazinring bilden; wenn A₁ eine lineare oder verzweigte, gesättigte oder ungesättigte Alkylen- oder Hydroxyalkylengruppe ist, kann B₁ auch eine Gruppe (CH₂)ₙ-CO-D-OC-(CH₂)ₙ- bedeuten,
worin D bedeutet:
(a) einen Glycolrest der Formel: -O-Z-O-, wobei Z eine lineare oder verzweigte Kohlenwasserstoffgruppe ist oder eine Gruppe bedeutet, die einer der folgenden Formeln entspricht:
-(CH2-CH2-O)x-CH2-CH2-
-[CH2-CH(CH3)-O]y-CH2-CH(CH3)-
wobei x und y eine ganze Zahl von 1 bis 4 bedeuten, die einen einzigen und wohldefinierten Polymerisationsgrad bedeutet, oder eine beliebige Zahl von 1 bis 4, die einen mittleren Polymerisationsgrad angibt;
(b) einen bis-sekundären Diaminrest, beispielsweise ein Piperazinderivat;
(c) einen bis-primären Diaminrest der Formel: -NH-Y-NH-, wobei Y eine lineare oder verzweigte Kohlenwasserstoffgruppe ist, oder die zweiwertige Gruppe
-CH₂-CH₂-S-S-CH₂-CH₂-;
(d) eine Ureylengruppe der Formel: -NH-CO-NH;
vorzugsweise ist X⁻ ein Anion;
(9) quartären Polyammoniumpolymeren, die Einheiten der folgenden Formel (VI) enthalten: 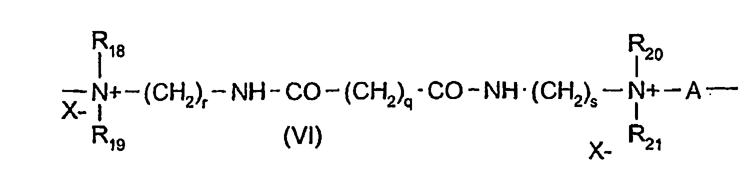 in der Formel:
R₁₈, R₁₉, R₂₀ und R₂₁, die identisch oder verschieden sind, bedeuten ein Wasserstoffatom oder eine Gruppe Methyl, Ethyl, Propyl, β-Hydroxyethyl, β-Hydroxypropyl oder -CH₂CH₂(OCH₂CH₂)ₚOH,
wobei p 0 ist oder eine ganze Zahl von 1 bis 6 bedeutet, mit der Maßgabe, dass die Gruppen R₁₈, R₁₉, R₂₀ und R₂₁ nicht gleichzeitig ein Wasserstoffatom bedeuten,
r und s, die gleich oder verschieden sind, sind ganze Zahlen im Bereich von 1 bis 6,
q bedeutet 0 oder eine ganze Zahl von 1 bis 34,
X ist ein Halogenatom,
A bedeutet eine Dihalogenidgruppe oder vorzugsweise
-CH₂-O-CH₂-CH₂-;
(10) quartären Polymeren von Vinylpyrrolidon und Vinylimidazol;
(11) Polyaminen, die nach CTFA-Lexikon unter dem Namen «POLYETHYLENEGLYCOL (15) TALLOW POLYAMINE» geführt werden;
(12) vernetzten Polymeren von Methacryloyloxyalkyl(C₁₋₄)trialkyl(C₁₋₄)ammoniumsalzen;
(13) Polyalkyleniminen, insbesondere Polyethyleniminen, Polymeren, die Vinylpyridin- oder Vinylpyridinium-Einheiten enthalten, Kondensaten von Polyaminen und Epichlorhydrin, quartären Polyureylenen und Chitinderivaten.

21. Zusammensetzung nach dem vorhergehenden Anspruch, **dadurch gekennzeichnet, dass** das kationische Polymer unter den kationischen Cyclopolymeren, kationischen Polysacchariden, quartären Polymeren von Vinylpyrrolidon und Vinylimidazol, vernetzten Homopolymeren oder Copolymeren von Methacryloyl-oxyalkyl(C₁₋₄)trialkyl(C₁₋₄)ammoniumsalzen, Polyalkyleniminen und deren Gemischen ausgewählt ist.

22. Zusammensetzung nach Anspruch 21, **dadurch gekennzeichnet, dass** das Cyclopolymer unter den Homopolymeren von Diallyldimethylammoniumchlorid und den Copolymeren von Diallyldimethylammoniumchlorid und Acrylamid ausgewählt ist.

23. Zusammensetzung nach Anspruch 21, **dadurch gekennzeichnet, dass** die kationischen Polysaccharide unter den Guargummen, die mit einem 2,3-Epoxypropyltrimethylammoniumsalz modifiziert sind, und den Hydroxyethylcellulosen ausgewählt sind, die mit einem mit einer Trimethylammoniumgruppe substituierten Epoxid umgesetzt wurden.

24. Zusammensetzung nach einem der Ansprüche 1 bis 18, **dadurch gekennzeichnet, dass** das amphotere Polymer ausgewählt ist unter:
(1) Polymeren, die bei der Copolymerisation eines Monomers, das von einer Vinylverbindung abgeleitet ist, die eine Carboxygruppe aufweist, wie insbesondere Acrylsäure, Methacrylsäure, Maleinsäure, α-Chloracrylsäure, und einem basischen Monomer gebildet werden, das von einer substituierten Vinylverbindung abgeleitet ist, die mindestens ein basisches Atom aufweist, wie insbesondere Dialkylaminoalkylmethacrylaten und Dialkylaminoalkylacrylaten, Dialkylaminoalkylmethacrylamiden und Dialkylaminoalkylacrylamiden; die Vinylverbindung kann auch ein Dialkyldiallylammoniumsalz sein, wie Dimethyldiallylammoniumchlorid;
(2) Polymeren, die Einheiten enthalten, die abgeleitet sind von:
a) mindestens einem Monomer, das unter den Acrylamiden oder Methacrylamiden ausgewählt ist, die am Stickstoff mit einer Alkylgruppe substituiert sind,
b) mindestens einem sauren Comonomer, das eine oder mehrere reaktive Carboxygruppen aufweist, und
c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfid oder Diethylsulfid;
(3) ganz oder teilweise alkylierten und vernetzten Polyaminoamiden, die von Polyaminoamiden der folgenden allgemeinen Formel abgeleitet sind:
⁅CO-R₄-CO-Z⁆ (VII)
in der R₄ eine zweiwertige Gruppe bedeutet, die von einer gesättigten Dicarbonsäure, einer aliphatischen Mono- oder Dicarbonsäure mit ethylenischer Doppelbindung, einem Ester dieser Säuren und einem niederen Alkanol mit 1 bis 6 Kohlenstoffatomen oder einer Gruppe abgeleitet ist, die durch Addition einer dieser Säuren und einem bis-primären oder bis-sekundären Amin gebildet wird, und Z bedeutet: eine bis-primäre, mono- oder bis-sekundäre Polyalkylenpolyamingruppe und vorzugsweise:
a) in Mengenanteilen von 60 bis 100 Mol-% die Gruppe der Formel (VIII) 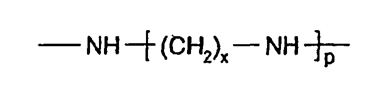 mit x=2 und p=2 oder 3 oder x=3 und p=2,
wobei diese Gruppe von Diethylentriamin, Triethylentetramin oder Dipropylentriamin abgeleitet ist;
b) in Mengenanteilen von 0 bis 40 Mol-% die oben angegebene Gruppe (IV) mit x=2 und p=1, die von Ethylendiamin stammt oder die von Piperazin abgeleitete Gruppe: 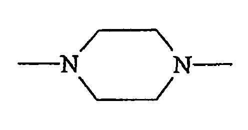
c) in Mengenanteilen von 0 bis 20 Mol-% die Gruppe -NH-(CH₂)₆-NH-, die von Hexamethylendiamin stammt, wobei diese Polyaminoamine durch Zugabe eines bifunktionellen Vernetzungsmittels, das unter den Epihalohydrinen, Diepoxiden, Dianhydriden, zweifach ungesättigten Derivaten ausgewählt ist, in einer Menge von 0,025 bis 0,35 mol Vernetzungsmittel pro Aminogruppe des Polyaminoamids vernetzt sind und durch Zugabe von Acrylsäure, Chloressigsäure oder eines Alkansultons oder ihren Salzen alkyliert sind;
(4) Polymeren, die zwitterionische Einheiten der Formel (IX) enthalten: 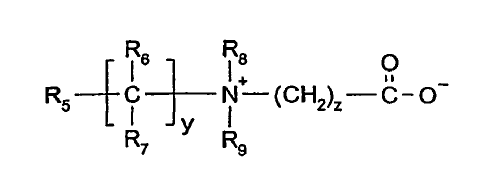 in der R₅ eine ungesättigte polymerisierbare Gruppe ist, beispielsweise eine Gruppe Acrylat, Methacrylat, Acrylamid oder Methacrylamid, y und z eine ganze Zahl von 1 bis 3 bedeuten, R₆ und R₇ ein Wasserstoffatom, Methyl, Ethyl oder Propyl bedeuten, R₈ und R₉ ein Wasserstoffatom oder eine Alkylgruppe bedeuten, wobei sie so gewählt sind, dass die Summe der Kohlenstoffatome in R₈ und R₉ 10 nicht übersteigt;
(5) Polymeren, die von Chitosan abgeleitet sind und Monomereinheiten enthalten, die den folgenden Formeln (X), (XI) und (XII) entsprechen: 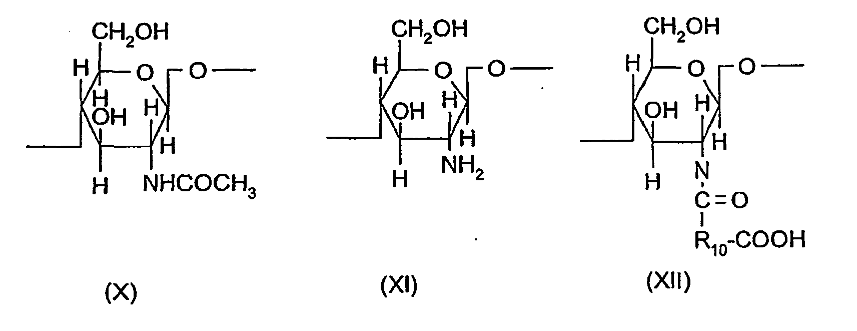 wobei die Einheit (X) in Mengenanteilen von 0 bis 30 % enthalten ist, die Einheit (XI) in Mengenanteilen von 5 bis 50 % enthalten ist und die Einheit (XII) in Mengenanteilen von 30 bis 90 % enthalten ist, mit der Maßgabe, dass in der Einheit (XII) die Gruppe R₁₀ eine Gruppe der folgenden Formel ist: 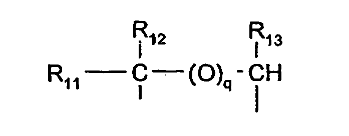 worin bedeuten:
wenn q=0, bedeuten die Gruppen R₁₁, R₁₂ und R₁₃, die gleich oder verschieden sind, jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und/oder gegebenenfalls mit einer oder mehreren Gruppen Amin, Hydroxy, Carboxy, Alkylthio, Sulfonsäure oder einem Alkylthiorest substituiert sind, dessen Alkylgruppe einen Aminorest trägt, wobei mindestens eine der Gruppen R₁₁, R₁₂ und R₁₃ in diesem Fall ein Wasserstoffatom ist;
oder wenn q=1, bedeuten die Gruppen R₁₁, R₁₂ und R₁₃ jede ein Wasserstoffatom, sowie die Salze, die diese Verbindungen mit Basen oder Säuren bilden;
(6) Polymeren, die durch N-Carboxyalkylierung von Chitosan gebildet werden, wie N-Carboxymethylchitosan oder N-Carboxybutylchitosan;
(7) Polymeren, die der allgemeinen Formel (XIII) entsprechen, wie den Polymeren, die beispielsweise in dem französischen Patent 1 400 366 beschrieben sind: 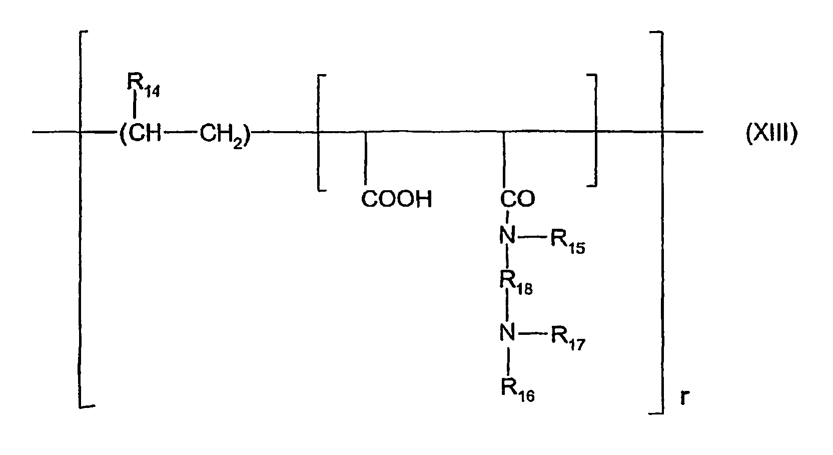 worin R₁₄ ein Wasserstoffatom, die Gruppe CH₃O, CH₃CH₂O, Phenyl bedeutet, R₁₅ Wasserstoff oder eine niedere Alkylgruppe wie Methyl, Ethyl ist, R₁₆ Wasserstoff oder eine niedere Alkylgruppe wie Methyl, Ethyl, bedeutet, R₁₇ eine niedere Alkylgruppe wie Methyl, Ethyl oder eine Gruppe ist, die der Formel: -R₁₈-N(R₁₆)₂ entspricht, wobei R₁₈ eine Gruppe -CH₂-CH₂-, -CH₂-CH₂-CH₂-, -CH₂CH(CH₃)- ist und R₁₆ die oben angegebenen Bedeutungen aufweist, sowie die höheren Homologen dieser Gruppen, die bis zu 6 Kohlenstoffatome enthalten;
(8) amphoteren Polymeren vom Typ -D-X-D-X-, die ausgewählt sind unter:
a) Polymeren, die durch Einwirkung von Chloressigsäure oder Natriumchloracetat auf Verbindungen erhalten werden, die mindestens eine Einheit der folgenden Formel aufweisen:
-D-X-D-X-D- (XIV)
wobei D die folgende Gruppe ist 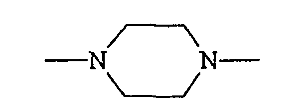 und X die Symbole E oder E' bedeutet, wobei E und E', die gleich oder verschieden sind, eine zweiwertige Gruppe bedeuten, bei der es sich um eine Alkylengruppe mit gerader oder verzweigter Kette handelt, die bis zu 7 Kohlenstoffatome in der Hauptkette aufweist und unsubstituiert vorliegt oder mit Hydroxygruppen substituiert ist und ferner Sauerstoffatome, Stickstoffatome, Schwefelatome, 1 bis 3 aromatische oder heterocyclische Ringe enthalten kann; wobei die Sauerstoffatome, Stickstoffatome und Schwefelatome in Form der Gruppen Ether, Thioether, Sulfoxid, Sulfon, Sulfonium, Alkylamin, Alkenylamin, Hydroxy, Benzylamin, Aminoxid, quartäres Ammonium, Amid, Imid, Alkohol, Ester und/oder Urethan vorliegen;
b) Polymeren der Formel:
-D-X-D-X- (XV)
wobei D die folgende Gruppe bedeutet 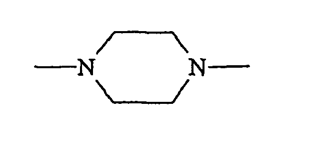 und X das Symbol E oder E' und mindestens einmal E' bedeutet; wobei E die oben angegebene Bedeutung hat und E' eine zweiwertige Gruppe ist, bei der es sich um eine Alkylengruppe mit gerader oder verzweigter Kette handelt, die bis zu 7 Kohlenstoffatome in der Hauptkette aufweist, unsubstituiert vorliegt oder mit einer oder mehreren Hydroxygruppen substituiert ist und ein oder mehrere Stickstoffatome aufweist, wobei das Stickstoffatom mit einer Alkylkette substituiert ist, die gegebenenfalls durch ein Sauerstoffatom unterbrochen ist und zwingend eine oder mehrere Carboxyfunktionen oder eine oder mehrere Hydroxyfunktionen aufweist und durch Umsetzung mit Chloressigsäure oder Natriumchloracetat betainisiert ist;
(9) Copolymeren Alkyl(C₁₋₅)vinylether/Maleinsäureanhydrid, das durch Semiamidierung mit einem N,N-Dialkyl-aminoalkylamin wie N,N-Dimethylaminopropylamin oder durch Semiveresterung mit einem N,N-Dialkanolamin partiell modifiziert ist.

25. Zusammensetzung nach einem der vorhergehenden Ansprüche, **dadurch gekennzeichnet, dass** das Copolymer von Methacrylsäure und Alkyl(C₁₋₄)acrylat in einer Konzentration von 0,01 bis 20 Gew.-%, bezogen auf das Gesamtgewicht der Zusammensetzung, und vorzugsweise im Bereich von 0,05 bis 15 Gew.-% enthalten ist.

26. Zusammensetzung nach einem der vorhergehenden Ansprüche, **dadurch gekennzeichnet, dass** die unlöslichen mineralischen Verbindungen in einer Konzentration von 0,001 bis 20 Gew.-%, bezogen auf das Gesamtgewicht der Zusammensetzung, und vorzugsweise im Bereich von 0,01 bis 10 Gew.-% vorliegen.

27. Zusammensetzung nach einem der vorhergehenden Ansprüche, **dadurch gekennzeichnet, dass** das kationische oder amphotere Polymer in einer Konzentration von 0,001 bis 20 Gew.-%, bezogen auf das Gesamtgewicht der Zusammensetzung, und vorzugsweise im Bereich von 0,01 bis 10 Gew.-% enthalten ist.

28. Zusammensetzung nach einem der Ansprüche 1 bis 27, **dadurch gekennzeichnet, dass** sie ferner mindestens ein Silicon enthält.

29. Zusammensetzung nach Anspruch 28, **dadurch gekennzeichnet, dass** die Silicone unter den nichtflüchtigen Polyorganosiloxanen, die ausgewählt sind unter den Polyalkylsiloxanen, Silicongummis und Siliconharzen, organomodifizierten Siliconen sowie deren Gemischen ausgewählt sind.

30. Zusammensetzung nach Anspruch 29, **dadurch gekennzeichnet, dass:**
(a) die Alkylsiloxane ausgewählt sind unter:
- Polydimethylsiloxanen mit endständigen Trimethylsilylgruppen;
- Polydimethylsiloxanen mit endständigen Dimethylsilanolgruppen;
- Polyalkyl(C₁₋₂₀)siloxanen;
(b) die Silicongummis ausgewählt sind unter den Polydiorganosiloxanen mit zahlenmittleren Molmassen im Bereich von 200 000 bis 1 000 000, die alleine oder in Form eines Gemisches in einem Lösungsmittel verwendet werden;
(d) die Harze ausgewählt sind unter den Harzen, die die folgenden Einheiten enthalten: R₃ Si O_{1/2}, R₂ Si O_{2/2}, R Si O_{3/2}, Si O_{4/2},
in denen R eine Kohlenwasserstoffgruppe mit 1 bis 16 Kohlenstoffatomen ist.

31. Zusammensetzung nach einem der Anspruche 28 bis 30, **dadurch gekennzeichnet, dass** die ergänzenden Silicone unter den Polyalkylsiloxanen mit endständigen Trimethylsilylgruppen, Polyalkylsiloxanen mit endständigen Dimethylsilanolgruppen, Gemischen aus zwei PDMS, die aus einem Gummi und einem Öl unterschiedlicher Viskositäten bestehen, Gemischen von Organosiloxanen und cyclischen Siliconen, Organopolysiloxanharzen ausgewählt sind.

32. Zusammensetzung nach einem der Ansprüche 1 bis 31, **dadurch gekennzeichnet, dass** sie ferner mindestens einen Stoff mit für Keratinsubstanzen günstiger Wirkung enthält, der unter den Estern von Carbonsäuren mit 1 bis 30 Kohlenstoffatomen und ein-oder mehrwertigen Alkoholen mit 1 bis 30 Kohlenstoffatomen, pflanzlichen Ölen, tierischen Ölen, Mineralölen oder synthetischen Ölen, Wachsen, Ceramiden, Pseudoceramiden ausgewählt ist.

33. Zusammensetzung nach einem der Ansprüche 28 bis 31, **dadurch gekennzeichnet, dass** das ergänzende Silicon in einer Konzentration im Bereich von 0,001 bis 20 Gew.-%, bezogen auf das Gesamtgewicht der Zusammensetzung, und vorzugsweise 0,01 bis 10 Gew.-% enthalten ist.

34. Zusammensetzung nach Anspruch 32, **dadurch gekennzeichnet, dass** der Stoff mit für Keratinsubstanzen günstiger Wirkung in einer Konzentration von 0,001 bis 20 Gew.-%, bezogen auf das Gesamtgewicht der Zusammensetzung, und vorzugsweise 0,01 bis 10 Gew.-% enthalten ist.

35. Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren, kationischen grenzflächenaktiven Stoffen und deren Gemischen ausgewählt ist.

36. Zusammensetzung nach Anspruch 35, **dadurch gekennzeichnet, dass** der oder die grenzflächenaktiven Stoffe ausgewählt sind unter mindestens einem oder mehreren anionischen grenzflächenaktiven Stoffen oder Gemischen von mindestens einem oder mehreren anionischen grenzflächenaktiven Stoffen und mindestens einem oder mehreren amphoteren grenzflächenaktiven Stoffen oder mindestens einem oder mehreren nichtionischen grenzflächenaktiven Stoffen.

37. Zusammensetzung nach einem der Ansprüche 35 bis 36, **dadurch gekennzeichnet, dass** der oder die grenzflächenaktiven Stoffe in einer Konzentration von 0,01 bis 50 Gew.-%, vorzugsweise 0,1 bis 40 Gew.-% und noch bevorzugter 0,5 bis 30 Gew.-%, bezogen auf das Gesamtgewicht der Zusammensetzung, vorliegen.

38. Zusammensetzung nach einem der vorhergehenden Ansprüche, **dadurch gekennzeichnet, dass** sie 3 bis 50 Gew.-% eines reinigenden grenzflächenaktiven Stoffes enthält.

39. Zusammensetzung nach einem der Ansprüche 1 bis 38, **dadurch gekennzeichnet, dass** die Zusammensetzung ferner mindestens einen Zusatzstoff enthält, der unter den Verdickungsmitteln, Antischuppenmitteln oder Wirkstoffen gegen Seborrhoe, Parfums, Perlglanzstoffen, Hydroxysäuren, Elektrolyten, Fettsäureestern, Konservierungsmitteln, siliconierten oder nichtsiliconierten Sonnenschutzfiltern, Vitaminen, Provitaminen wie Panthenol, anionischen oder nichtionischen Polymeren, Proteinen, Proteinhydrolysaten, 18-Methyleicosansäure, fluorierten oder perfluorierten Ölen, Fettaminen, Fettsäuren und ihren Derivaten, Fettalkoholen und ihren Derivaten sowie den Gemischen dieser verschiedenen Verbindungen ausgewählt ist.

40. Zusammensetzung nach einem der vorhergehenden Ansprüche, **dadurch gekennzeichnet, dass** sie als Haarwaschmittel, Produkt, das nach der Haarwäsche aufgebracht wird, Zusammensetzung für dauerhafte Verformungen, Entkräuselungen, Färbungen oder Entfärbungen der Haare, Zusammensetzung, die ausgespült wird und zwischen den beiden Schritten einer permanenten Verformung oder Entkräuselung aufgebracht wird, reinigende Zusammensetzung für den Körper vorliegt.

41. Verwendung einer Zusammensetzung nach einem der vorhergehenden Ansprüche für die Reinigung oder für die Pflege von Keratinsubstanzen.

42. Verfahren zur Behandlung von Keratinsubstanzen wie Haaren, **dadurch gekennzeichnet, dass** es darin besteht, auf die Keratinsubstanzen eine kosmetische Zusammensetzung nach einem der Ansprüche 1 bis 40 aufzubringen und anschließend gegebenenfalls mit Wasser zu spülen.

43. Verwendung eines vernetzten Methacrylsäure/Alkyl(C₁₋₄)acrylat-Copolymers in einer kosmetischen Zusammensetzung oder für die Herstellung einer kosmetischen Zusammensetzung, die ein kationisches oder amphoteres Polymer und zumindest in Wasser unlösliche feste Mineralpartikel, die unter den Tonen ausgewählt sind, Partikel, die im Wesentlichen aus Aluminiumoxid bestehen, Partikel, die mindestens 10 Gew.-% Calciumcarbonat enthalten, und Selensulfid enthält.

44. Verwendung einer Zusammensetzung nach einem der Ansprüche 1 bis 40, um dem Haar Weichheit zu geben.
